(19) Europäisches Patentamt
European Patent Office
Office européen des brevets

(11) EP 3 161 026 B1

(12) FASCICULE DE BREVET EUROPEEN

(45) Date de publication et mention de la délivrance du brevet:
**10.10.2018 Bulletin 2018/41**

(21) Numéro de dépôt: **15732230.6**

(22) Date de dépôt: **25.06.2015**

(51) Int Cl.:
***C08F 220/18*** (2006.01) ***A61K 8/81*** (2006.01)
***A61Q 19/08*** (2006.01) ***C08L 33/08*** (2006.01)
***C08L 33/10*** (2006.01) ***C08L 43/02*** (2006.01)

(86) Numéro de dépôt international:
**PCT/EP2015/064429**

(87) Numéro de publication internationale:
**WO 2015/197778 (30.12.2015 Gazette 2015/52)**

(54) **COPOLYMÈRE PHOSPHONIQUE ET SON UTILISATION EN COSMÉTIQUE**

PHOSPHONCOPOLYMER UND DESSEN VERWENDUNG IM KOSMETISCHEN BEREICH

PHOSPHONIC COPOLYMER AND USE THEREOF IN THE COSMETICS FIELD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **25.06.2014 FR 1455877**

(43) Date de publication de la demande:
**03.05.2017 Bulletin 2017/18**

(73) Titulaire: **L'Oréal**
**75008 Paris (FR)**

(72) Inventeurs:
- **LION, Bertrand**
  **93601 Aulnay-sous-Bois (FR)**
- **SABATIE, Laurent**
  **La Varenne Saint Hilaire 94210 (FR)**

(74) Mandataire: **Kromer, Christophe**
**L'Oréal**
**Service DIPI**
**9 Rue Pierre Dreyfus**
**92110 Clichy (FR)**

(56) Documents cités:
**EP-A1- 1 621 560**
**EP-A1- 1 800 659**
**EP-A2- 1 776 946**
**US-B1- 6 268 454**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 161 026 B1

## Description

La présente invention concerne de nouveaux polymères à groupe acide phosphonique et leur utilisation dans le domaine de la cosmétique.

Au cours du processus de vieillissement, il apparaît différents signes sur la peau, très caractéristiques de ce vieillissement, se traduisant notamment par une modification de la structure et des fonctions cutanées. Les principaux signes cliniques du vieillissement cutané sont notamment l'apparition de ridules et de rides profondes, en augmentation avec l'âge.

Il est connu de traiter ces signes du vieillissement en utilisant des compositions cosmétiques ou dermatologiques contenant des actifs capables de lutter contre le vieillissement, tels que les $\alpha$-hydroxy-acides, les $\beta$-hydroxy-acides et les rétinoïdes. Ces actifs agissent sur les rides en éliminant les cellules mortes de la peau et en accélérant le processus de renouvellement cellulaire. Toutefois, ces actifs présentent l'inconvénient de n'être efficaces pour le traitement des rides qu'après un certain temps d'application. Or, on cherche de plus en plus à obtenir un effet immédiat des actifs utilisés, conduisant rapidement à un lissage des rides et ridules et à la disparition des marques de fatigue.

EP1776946 décrit une composition cosmétique anti-rides comprenant, dans un milieu physiologiquement acceptable, au moins un copolymère statistique à chaîne principale linéaire de nature éthylénique, dans laquelle ledit copolymère consiste en au moins 70 % en poids d' unités monomères issues de monomères dont les homopolymères sont hydrophobes et présentent une température de transition vitreuse supérieure à 40°C et au moins une unité monomère issue d'un monomère hydrophile ionique au moins partiellement neutralisée.

EP1621560 divulgue un copolymère comprenant au moins deux branches polymériques, qui comportent, chacune, au moins un point de ramification au moins trivalent, distinct et indépendant l'un de l'autre caractérisé en ce que une première branche comprend au moins un premier monomère sélectionné, choisi dans le groupe formé par l'acrylate d'isobomyle, le méthacrylate d'isobomyle, l'acrylate d'isobutyle, le méthacrylate d'isobutyle, et l'acrylate d'éthyle-2-hexyle et en ce que au moins une deuxième branche comprend au moins un deuxième monomère sélectionné, identique ou différent dudit premier monomère, et choisi dans ledit groupe. Le copolymère peut aussi comprendre 0 à 50% en poids d'un monomère additionnel.

Les inventeurs ont découvert que de nouveaux polymères à groupe acide phosphonique permettent d'obtenir un film transparent (non blanchâtre) et homogène, qui ne s'effrite pas, contrairement aux copolymères similaires contenant de l'acide acrylique à la place du monomère acide phosphonique. Le film présente en outre la propriété de ne pas coller ni de transférer au contact du doigt.

De tels polymères présentent également un effet tenseur sur la peau amélioré et permet ainsi d'atténuer les rides de la peau de façon immédiate. De plus, lorsque le polymère phosphonique est associé avec un composé aminé particulier, l'effet tenseur obtenu est encore amélioré et cet effet présente en plus une bonne résistance à l'eau et donc une bonne rémanence à l'eau.

Ces polymères filmogènes conviennent également pour le maquillage des matières kératiniques, en particulier pour le maquillage de la peau ou des lèvres tels que les fonds de teint, les rouges à lèvres.

De façon plus précise, la présente invention a pour objet un copolymère issu de la polymérisation de :

(i) 70 à 95 % en poids, du poids total des monomères, de (méth)acrylate d'isobornyle ;
(ii) 1 à 30 % en poids de monomère acide vinyl phosphonique de formule (I) défini ci-après ;
(iii) 0 à 20 % en poids d'un monomère additionnel.

Un tel copolymère est appelé par la suite polymère phosphonique.

L'invention a également pour objet une composition comprenant, dans un milieu physiologiquement acceptable, un polymère phosphonique tel que décrit précédemment.

L'invention a également pour objet un procédé, notamment cosmétique, de soin ou de maquillage des matières kératiniques, en particulier de la peau, comprenant l'application topique sur les matières kératiniques, en particulier de la peau, d'une composition, notamment cosmétique, comprenant un polymère phosphonique tel que décrit précédemment.

L'invention a en particulier pour objet un procédé, notamment cosmétique, de soin ou de maquillage de la peau ou des lèvres, plus particulièrement de la peau du visage, en particulier de la peau ridée, comprenant l'application topique sur la peau d'une composition, notamment cosmétique, comprenant un polymère phosphonique tel que décrit précédemment.

L'invention a également pour objet un procédé, notamment cosmétique, de soin de la peau, plus particulièrement de la peau du visage, en particulier de la peau ridée, comprenant l'application topique sur la peau d'une composition, notamment cosmétique, comprenant un polymère phosphonique tel que décrit précédemment.

Le procédé selon l'invention est en particulier destiné à lisser la peau humaine du visage et/ou du corps et/ou à diminuer ou effacer les signes du vieillissement cutané, en particulier à réduire ou effacer les rides et/ou les ridules

de la peau.

Selon un mode de réalisation du procédé selon l'invention, on effectue l'application topique sur les matières kératiniques, en particulier sur la peau, d'un mélange extemporané d'une composition comprenant un polymère phosphonique telle que décrite précédemment et d'un composé aminé, ou d'une composition le contenant et comprenant un milieu physiologiquement acceptable, tel que défini ci-après.

Selon un autre mode de réalisation du procédé selon l'invention, on effectue l'application séquentielle sur les matières kératiniques, en particulier sur la peau, d'une composition comprenant un polymère phosphonique telle que décrite précédemment et d'un composé aminé, ou d'une composition le contenant et comprenant un milieu physiologiquement acceptable, tels que définis ci-après.

L'invention a également pour objet l'utilisation cosmétique en tant qu'agent tenseur de la peau, en particulier d'une peau ridée, d'un polymère phosphonique tel que décrit précédemment, éventuellement en mélange avec un composé aminé, ou d'une composition le contenant et comprenant un milieu physiologiquement acceptable, tel que défini ci-après.

L'invention a aussi pour objet une composition, notamment cosmétique, obtenue par mélange d'une composition comprenant ledit polymère phosphonique telle que décrite précédemment et d'un composé aminé ou d'une composition le contenant et comprenant un milieu physiologiquement acceptable, tel que défini ci-après.

L'invention a encore pour objet un kit comprenant une première composition comprenant ledit polymère phosphonique telle que décrite précédemment et une deuxième composition comprenant un composé aminé tel que décrit ci-après et comprenant un milieu physiologiquement acceptable, les première et deuxième compositions étant conditionnées chacune dans un ensemble de conditionnement distinct.

L'ensemble de conditionnement des compositions est de façon connue tout packaging adapté pour stocker les compositions cosmétiques (flacons, tube, flacon spray, flacon aérosol notamment).

Un tel kit permet de mettre en oeuvre le procédé de traitement de la peau selon l'invention.

Le polymère phosphonique selon l'invention comprend un (méth)acrylate d'isobornyle, un monomère acide vinyl phosphonique de formule (I) et éventuellement un monomère additionnel tels que définis ci-après.

Le monomère acide vinyl phosphonique répond à la formule (I) suivante :

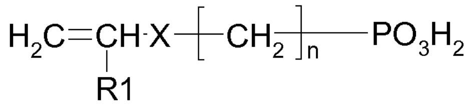

(I)

dans laquelle :

R1 désigne H ou $-CH_3$ ;
X désigne une liaison covalente et n désigne un nombre entier allant de 0 à 14 ;
ou X désigne un groupement -COO- et n désigne un nombre entier allant de 2 à 6.

Avantageusement, pour le monomère de formule (I), X désigne une liaison covalente et n est un nombre entier allant de 0 à 6 ou X désigne un groupe -COO- et n est un nombre entier allant de 2 à 4.

Préférentiellement, pour le monomère de formule (I) :

R1 = H

X désigne une liaison covalente et n est un nombre entier allant de 0 à 4.

Comme exemple de monomère de formule (I), on peut citer :

l'acide vinyl phosphonique ;
l'acide 3-butenyl phosphonique ;
l'acide 4-pentenyl phosphonique ;
l'acide 10-undecenyl phosphonique ;
l'acide 11-dodecenyl phosphonique ;
l'ester 2-phosphonoethyl de l'acide 2- propenoïque ;
l'ester 2-phosphonoethyl de l'acide 2-methyl 2- propenoïque.

Préférentiellement, le monomère (I) est l'acide vinyl phosphonique.

Le monomère additionnel éventuellement présent est différent des monomères de (méth)acrylate d'isobornyle et d'acide vinyl phosphonique.

En particulier, le monomère additionnel peut être choisi parmi les monomères de formule (II) :

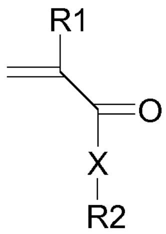

(II)

dans laquelle :

$R_1$ désigne un atome d'hydrogène ou un radical méthyle ;
X désigne O ou NH ou $NR_3$ ;
$R_2$ désigne ou radical alkyl linéaire en $C_1$-$C_{22}$ ou ramifié en $C_3$-$C_{10}$ ou cyclique en $C_5$-$C_7$ ou un radical hydrocarboné insaturé linéaire en $C_3$-$C_{20}$ ou ramifié en $C_6$-$C_{20}$ ou cyclique en $C_5$-$C_7$ ou un radical $-(Si(CH_3)_2O)_b-CH_3$ avec b allant de 5 à 70, étant entendu que X = O lorsque $R_2$ est un radical de formule-$(Si(CH_3)_2O)_b-CH_3$ ;
$R_3$ désigne un radical alkyle linéaire en $C_1$-$C_{12}$ ou ramifié en $C_3$-$C_{12}$.

Comme radical alkyle on peut citer les radicaux méthyle, éthyle, propyle, isopropyle, butyle, tertiobutyle, pentyle, isopentyle, hexyle, heptyle, octyle, isooctyle, nonyle, décyle, undécyle,.lauryle, myristyle, palmityle, stéaryle, eicosyle, béhényle.
Selon un mode de réalisation préféré, le copolymère est exempt de monomère additionnel.
Avantageusement, on utilise des copolymères de (méth)acrylate d'isobornyle et d'acide vinylphosphonique.
Le polymère selon l'invention est de préférence choisi parmi les copolymères issus de la polymérisation de :

- 70 à 95 % en poids, du poids total des monomères, de (méth)acrylate d'isobornyle ;
- 5 à 30 % en poids de monomère acide vinyl phosphonique de formule (I) tel que décrit précédemment.

Préférentiellement, le polymère selon l'invention est choisi parmi les copolymères issus de la polymérisation de :

- 75 à 95 % en poids, du poids total des monomères, de (méth)acrylate d'isobornyle ;
- 5 à 25 % en poids de monomère acide vinyl phosphonique de formule (I) tel que décrit précédemment.

Selon un mode préféré de l'invention, le polymère est choisi parmi les copolymères issus de la polymérisation de :

- 70 à 95 % en poids, du poids total des monomères, de (méth)acrylate d'isobornyle ;
- 5 à 30 % en poids d'acide vinyl phosphonique ;

et préférentiellement, de :

- 75 à 95 % en poids, du poids total des monomères, de (méth)acrylate d'isobornyle ;
- 5 à 25 % en poids d'acide vinyl phosphonique.

Comme exemples de copolymères selon l'invention on peut citer les copolymères acrylate d'isobornyle / acide vinyl phosphonique, en particulier les copolymères acrylate d'isobornyle / acide vinylphosphonique (80/20) ou 90/10 (poids/poids).
Le copolymère peut être un polymère statistique, alterné (bloc), à gradient. De préférence, le copolymère est statistique.
Le copolymère selon l'invention peut être préparé par polymérisation radicalaire des monomères décrits précédemment, notamment en mélange ou ajoutés séquentiellement pendant la polymérisation, notamment en utilisant un solvant organique ayant un point d'ébullition supérieur ou égal à 60 °C, comme par exemple l'isododécane, l'éthanol, l'acétate d'éthyle, le tétrahydrofurane, le méthyltétrahydrofurane, la méthyl éthtyl cétone. Le solvant organique permet de solubiliser les monomères mis en oeuvre et le polymère formé.
La polymérisation est notamment effectuée en présence d'un amorceur radicalaire notamment de type peroxyde (par

exemple tert-Butyl peroxy-2-ethylhexanoate : Trigonox 21S ; 2,5-dimethyl-2,5-di(2-ethylhexanoylperoxy)hexane :Trigonox 141 ; tert-butyl peroxypivalate : Trigonox 25C75 de chez AkzoNobel) ou azoïque par exemple (AIBN : azobisisobutyronitrile ; V50 : 2,2'-azo-bis(2-amidinopropane) dihydrochlorure).

La polymérisation peut s'effectuer à une température allant de 60 à 100 °C, de préférence allant de 60 à 85 °C. La durée de la polymérisation peut être d'environ 24 heures.

On entend par « agent tenseur » des composés susceptibles d'avoir un effet tenseur apparent, c'est-à-dire de lisser la peau et réduire, voire faire disparaître, de façon immédiate les rides et les ridules.

L'effet tenseur peut être caractérisé par un test *in vitro* de rétractation tel que décrit à l'exemple 5.

De préférence, le polymère phosphonique selon l'invention a un poids moléculaire moyen en poids allant de 5000 à 1 000 000 daltons, plus préférentiellement allant de 10000 à 500 000 daltons, et encore plus préférentiellement allant de 15000 à 350 000 daltons. Le poids moléculaire peut notamment être déterminé par chromatogaphie par exclusion stérique , éluant THF + 0,2 M LiCl, étalon polystyrène, détecteur refractomètre 2414 de chez WATERS.

Le polymère phosphonique tel que défini précédemment peut être présent dans la composition selon l'invention en une teneur allant de 0,1 à 10 % en poids, par rapport au poids total de la composition, de préférence de 0,5 % à 10 % en poids, et préférentiellement allant de 1 % à 8 % en poids, et plus préférentiellement allant de 1 % à 6 % en poids.

Selon un premier mode de réalisation du procédé selon l'invention, on réalise un mélange extemporané du polymère phosphonique selon l'invention et d'un composant additionnel tel que défini ci-après et le mélange est appliqué sur la peau.

Selon un deuxième mode de réalisation du procédé selon l'invention, on effectue une application séquentielle d'une part du polymère phosphonique et d'autre part d'un composant additionnel tel que défini ci-après.

Le composant additionnel mis en oeuvre dans le procédé selon l'invention est notamment un composé aminé choisi parmi les composés aminés ayant plusieurs groupes amine primaire et/ou amine secondaire ou bien encore les aminosilanes. Il peut donc être choisi parmi les composés aminosilanes, les composés diaminés, les composés triaminés ou les composés polyaminés.

Selon un premier mode de réalisation de l'invention, le composé aminé est un composé comprenant de 2 à 20 atomes de carbones, notamment un composé non polymérique. Par composé non polymérique, on entend un composé qui n'est pas directement obtenu par une réaction de polymérisation de monomères.

Comme composés aminés, on peut citer le N-méthyl-1,3-diaminopropane, le N-propyl 1,3-diaminopropane, le N-isopropyl 1,3-diaminopropane, le N-cyclohexyl 1,3-diaminopropane, le 2-(3-aminopropylamino) éthanol, le 3-(2-aminoéthyl)aminopropylamine, le bis(3-aminopropyl)amine, la méthyl bis(3-aminopropyl)amine, le N-(3-aminopropyl)-1,4-diaminobutane, la N,N-diméthyldipropylène triamine, le 1,2-bis(3-aminopropylamino)éthane, la N,N'-bis(3-aminopropyl)-1,3-propanediamine, l'éthylène diamine, la 1,3-propylènediamine, la 1,4-butylènediamine, la lysine, la cystamine , la xylène diamine, la tris(2-aminoéthyl)amine, la spermidine.

Le composé aminé peut être également choisi parmi les aminosilanes, tels que ceux de formule (III) :

$$R'_1Si(OR'_2)_z(R'_3)_x \qquad (III)$$

dans laquelle :

- $R'_1$ est une chaîne hydrocarbonée en $C_1$-$C_6$, linéaire ou ramifiée, saturée ou insaturée, cyclique ou acyclique substituée par un groupement choisi parmi les groupements :
  - amine $NH_2$ ou NHR avec R= alkyle en $C_1$-$C_4$,
  - un groupement aryle ou aryloxy substitué par un groupement amino ou par un groupement aminoalkyl en $C_1$-$C_4$; $R'_1$ pouvant être interrompu dans sa chaine par un hétéroatome (O, S, NH) ou un groupement carbonyle (CO), $R'_1$ étant lié à l'atome de silicium directement via un atome de carbone,
- $R'_2$ et $R'_3$ identiques ou différents, représentent un groupe alkyle, linéaire ou ramifié, comprenant de 1 à 6 atomes de carbone,
- z désigne un nombre entier allant de 1 à 3, et
- x désigne un nombre entier allant de 0 à 2,

avec z+x =3.

De préférence, $R'_2$ représente un groupe alkyle comprenant de 1 à 4 atomes de carbone.
De préférence, $R'_2$ représente un groupe alkyle linéaire comprenant de 1 à 4 atomes de carbone.
De préférence, $R'_2$ représente le groupe éthyle.

De préférence, $R'_3$ représente un groupe alkyle comprenant de 1 à 4 atomes de carbone.

De préférence, $R'_3$ représente un groupe alkyle linéaire comprenant de 1 à 4 atomes de carbone.

De préférence, $R'_3$ représente le groupe méthyle ou éthyle.

De préférence $R'_1$ est une chaîne acyclique.

De préférence $R'_1$ est une chaîne hydrocarbonée en $C_1$-$C_6$, linéaire ou ramifiée, saturée ou insaturée, substituée par un groupement amine $NH_2$ ou NHR (R= alkyle en $C_1$-$C_6$, cycloalkyle en $C_3$-$C_6$ ou aromatique en $C_6$). Préférentiellement, $R'_1$ est une chaîne hydrocarbonée en $C_1$-$C_6$, linéaire saturée substituée par un groupement amine $NH_2$. Plus préférentiellement, $R'_1$ est une chaîne hydrocarbonée en $C_2$-$C_4$, linéaire saturée substituée par un groupement amine $NH_2$.

De préférence, $R'_1$ est une chaîne hydrocarbonée en $C_1$-$C_6$, linéaire saturée substituée par un groupement amine $NH_2$,
$R'_2$ représente un groupe alkyle comprenant de 1 à 4 atomes de carbone,
$R'_3$ représente un groupe alkyle comprenant de 1 à 4 atomes de carbone.

De préférence z est égal à 3.

De préférence, l'aminosilane de formule (III) est choisi parmi le 3-aminopropyltriéthoxysilane (APTES), le 3-aminoéthyltriéthoxysilane (AETES), le 3-aminopropylméthyldiéthoxysilane, le N-(2-aminoéthyl)-3-aminopropyltriéthoxysilane, le 3-(m-aminophénoxy)propyltriméthoxysilane, le p-aminophényltriméthoxysilane, le N-(2-aminoéthylaminométhyl)phénéthyltriméthoxysilane.
De préférence, l'aminosilane (III) est choisi parmi le 3-aminopropyltriéthoxysilane (APTES), le 3-aminoéthyltriéthoxysilane (AETES), le 3-aminopropylméthyldiéthoxysilane, le N-(2-aminoéthyl)-3-aminopropyltriéthoxysilane.
De préférence l'aminosilane (III) est le 3-aminopropyl triéthoxysilane (APTES).

De préférence, le composé aminé est choisi parmi le 3-aminopropyltriethoxysilane (APTES), , le N-méthyl-1,3-diaminopropane, le N-propyl 1,3-diaminopropane, le N-isopropyl 1,3-diaminopropane, le N-cyclohexyl 1,3-diaminopropane, le 2-(3-aminopropylamino) éthanol, le 3-(2-aminoéthyl)aminopropylamine, le bis(3-aminopropyl)amine, la méthyl bis(3-aminopropyl)amine, le N-(3-aminopropyl)-1,4-diaminobutane, la N,N-diméthyldipropylène triamine, le 1,2-bis(3-aminopropylamino)éthane, la N,N'-bis(3-aminopropyl)-1,3-propanediamine, l'éthylène diamine, la lysine.

Le composé aminé peut également être choisi parmi les polymères aminés, notamment ayant un poids moléculaire moyen en poids allant de 500 à 1 000 000, de préférence allant de 500 à 500 000, et préférentiellement allant de 500 à 100 000.

Comme polymère aminé on peut utiliser les poly(alkylène ($C_2$-$C_5$) imines), et en particulier les polyéthylèneimines et les polypropylèneimines, notamment les poly(éthylène imine) (par exemple celui vendu sous la référence 46,852-3 par la société Aldrich Chemical) ; la poly(allylamine) (par exemple celle vendue sous la référence 47,913-6 par la société Aldrich Chemical) ; les polyvinylamines et leurs copolymères notamment avec des vinylamides; on peut notamment citer les copolymères vinylamine/vinylformamide tels que ceux commercialisés sous la dénomination LUPAMIN® 9030 par la société BASF ; les polyacides aminés présentant des groupes $NH_2$ comme la polylysine, par exemple celle vendu par la société JNC Corporation (anciennement Chisso) ; l'amino dextrane, tel que celui vendu par la société CarboMer Inc ; l'amino alcool polyvinylique tel que celui vendu par la société CarboMer Inc, les copolymères à base d'acrylamidopropylamine; les chitosanes ;
Les polydiméthylsiloxanes comprenant des groupes amines primaires en bout de chaine ou sur des chaines latérales, par exemple des groupes terminaux ou latéraux aminopropyl, comme par exemple ceux de formule (A) ou (B) ou (C) :

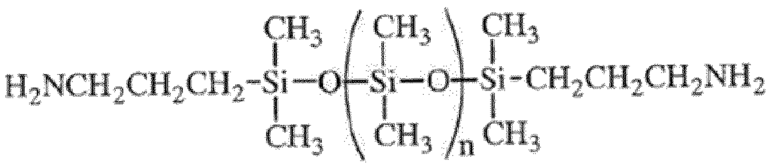

(A)

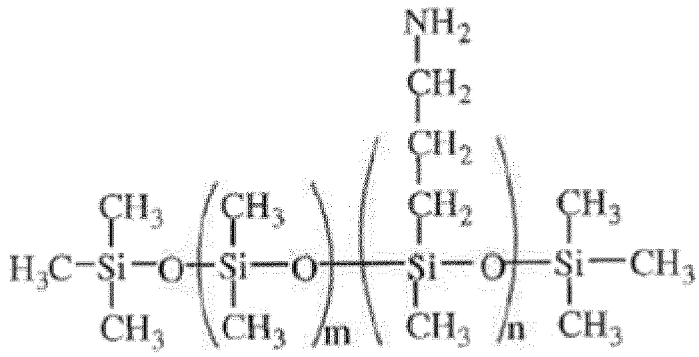

(B)

$H_2NCH_2CH_2CH_2\text{-}Si(CH_3)_2\text{-}O\text{-}[Si(CH_3)_2\text{-}O]_n\text{-}Si(CH_3)_2C_4H_9$ (C)

dans la formule (A) : la valeur de n est telle que le poids moléculaire moyen en poids de la silicone est compris entre 500 et 55 000. Comme exemple de silicone aminée (A) on peut citer celles vendues sous les dénominations « DMS-A11 », « DMS-A12 », « DMS-A15 », « DMS-A21 », « DMS-A31 », « DMS-A32, « DMS-A35 » par la société GELEST. dans la formule (B), les valeurs de n et m sont telles que le poids moléculaire moyen en poids de la silcione est compris entre 1000 et 55 000. Comme exemples de silicone (B) on peut citer celles vendues sous les dénominations «AMS-132 », « AMS-152 », « AMS-162 », « AMS-163 », « AMS-191 », «AMS-1203 » par la société GELEST. dans la formule (C), la valeur de n est telle que le poids moléculaire moyen en poids de la silicone est compris entre 500 et 3000. Comme exemple de silicone (C), on peut citer celles vendues sous les dénominations « MCR-A11 », « MCR-A12 » par la société GELEST.
les amodiméthicones de formule (D) :

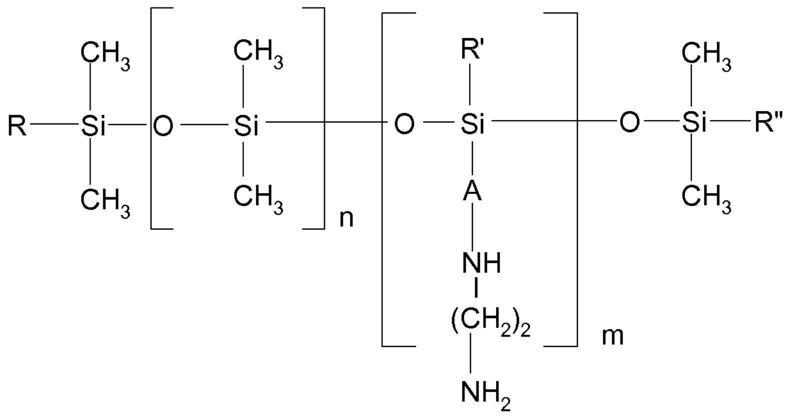

(D)

dans laquelle R, R' et R", identiques ou différents, représentent chacun un groupe alkyle en $C_1$-$C_4$ ou hydroxyle, A représente un groupe alkylène en $C_3$ et m et n sont tels que la masse moléculaire moyenne en poids du composé est comprise entre 5 000 et 500 000 environ.
Les polyéthers amines notamment connues sous la référence JEFFAMINE de la société HUNSTMAN ; et notamment :
Les polyéthylèneglycol et/ou polypropylèneglycol $\alpha$, $\omega$-diamine (à fonction amine en bout de chaine) comme celles vendues sous les dénominations JEFFAMINE D-230, D-400, D-2000, D-4000, ED-600, ED-9000, ED-2003.
Les polytétrahydrofurane (ou polytétraméthylèneglycol) $\alpha$, $\omega$-diamine ,
les polybutadiènes $\alpha$, $\omega$-diamine ;
Les dendrimères polyamidoamine (PANAM) à fonctions amines terminales.

Les poly(méth)acrylates ou poly(méth)acrylamides porteurs de fonctions amines primaires ou secondaires latérales telles que le poly(3-aminopropyl)méthacrylamide, le poly(2-aminoéthyl) méthacrylate.

Comme polymère aminé, on utilise de préférence les polyéthylèneglycol et/ou polypropylèneglycol $\alpha$, $\omega$-diamine et les polydiméthylsiloxanes comprenant des groupes terminaux aminopropyl,
Préférentiellement, les composés aminés utilisés dans le procédé selon l'invention sont choisis parmi l'éthylène diamine, la lysine, le 3-aminopropyltriethoxy-silane (APTES). Plus préférentiellement, on utilise le 3-aminopropyltriethoxy-silane

(APTES).

Avantageusement, le composé aminé utilisé dans le procédé selon l'invention est mis en oeuvre selon un ratio molaire composé aminé / acide phosphonique allant de 0,01 à 10, de préférence allant de 0,1 à 5, préférentiellement allant de 0,1 à 2, et plus préférentiellement allant de 0,1 à 1.

Le composé aminé au contact avec le polymère phosphonique réagit avec les fonctions acide phosphonique, par exemple de la façon suivante :

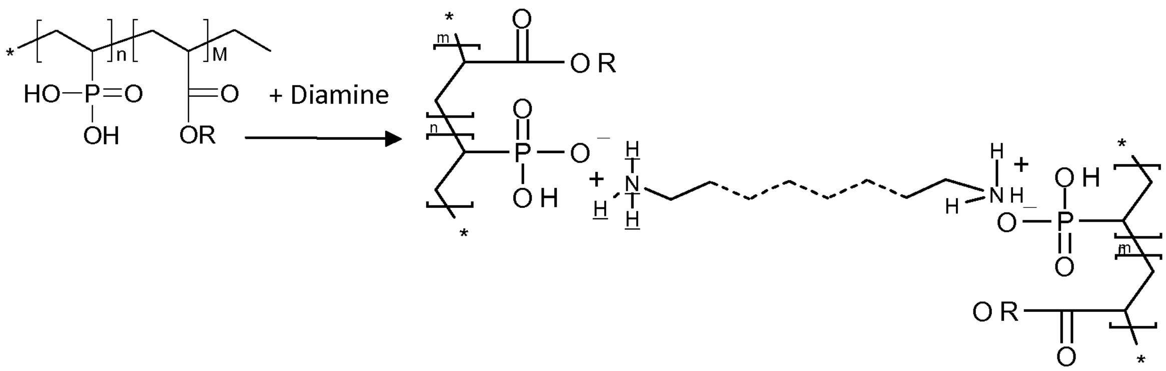


D'autres composants additionnels particuliers peuvent être mis en oeuvre dans le procédé selon l'invention pour contribuer à améliorer les propriétés filmogène du polymère selon l'invention. De tels composants additionnels sont notamment les sels d'ions de métal divalent ou trivalent, les argiles, les oxydes métalliques décrits ci-après.

La composition selon l'invention peut comprendre des sels d'ions de métal divalent ou trivalent , en particulier choisis parmi les sels d'ions issus de Al(III), Ca(II), Cu(II), Fe(II), Fe(III), Mg(II), Mn(II), Zn(II) et leurs mélanges. Les ions issus de Ca(II), Mg(II) sont préférés.

Les sels de ces ions métalliques sont bien connus, avec par exemples les anions tels que gluconate, chlorure, sulfate, hydroxyde , acétate, stéarate . Par exemple, on peut utiliser les sels suivants : gluconate de calcium, chlorure de calcium, chlorure de magnésium, chlorure de cuivre, gluconate de magnésium , le sulfate de fer, gluconate de fer, sulfate d'aluminium, stéarate de sodium.

Le dit sels d'ions de métal divalent ou trivalent peut être présent dans la composition selon l'invention en une teneur allant de 0,1 à 20 % en poids, de préférence de 0,1 à 15 % en poids, par rapport au poids total de la composition.

Alternativement, le sel d'ions de métal divalent ou trivalent peut être appliqué séquentiellement dans le procédé selon l'invention

La composition selon l'invention peut comprendre une argile.

Les argiles sont des produits déjà bien connus en soi, qui sont décrits par exemple dans l'ouvrage "Minéralogie des argiles, S. Caillère, S. Hénin, M. Rautureau, 2ème édition 1982, Masson", dont l'enseignement est ici inclus à titre de référence.

Parmi les argiles, on peut citer à titre d'exemples les argiles de la famille des smectites telles que la laponite et la montmorillonite, de la famille des kaolinites telles que la kaolinite, la dickite, la nacrite, les argiles éventuellement modifiées de la famille de l'halloysite, de la dombassite, de l'antigorite, de la benthiérine, de la pyrophyllite, des montmorillonites, de la beidellite, des vermiculites, du talc, de la stévensite, des hectorites, des bentonites, des saponites, des chlorites, de la sépiolite et de l'illite.

La ou les argiles présentes dans la composition de l'invention peuvent être naturelles ou de synthèse. L'argile naturelle est une roche sédimentaire composée pour une large part de minéraux spécifiques, silicates en général d'aluminium. Le kaolin est ainsi une argile naturelle.

Les argiles peuvent également être modifiées chimiquement par divers composés tels que les acides acryliques, les polysaccharides (par exemple la carboxyméthylcellulose) ou les cations organiques.

De préférence, on utilise dans le cadre de la présente invention les argiles qui sont cosmétiquement compatibles avec et acceptables pour les cheveux, la peau et / ou le cuir chevelu.

Selon un mode de réalisation particulier de la présente invention, l'argile mise en oeuvre est choisie parmi la kaolinite, les montmorillonites, les saponites, les laponites, les bentonites, et en particulier les hectorites, et les illites. On utilise encore plus particulièrement les mélanges d'argiles, et des argiles naturelles.

A titre d'argile naturelle, on peut citer les argiles vertes, notamment riches en illite ; les argiles riches en montmorillonite, connues sous l'appellation terre de Sommière, ou comme les bentonites, ou encore les argiles blanches riches en kaolinite. Comme bentonites, on peut en particulier citer celles commercialisées sous les dénominations "BENTONE 38 VCG", "BENTONE GEL CAO V", "BENTONE 27 V" et "BENTONE GEL MIO V" « BENTONE GEL ISD V » par la société ELEMENTIS.

Les montmorillonites et les smectites sont des silicates d'aluminium et / ou de magnésium hydratés. On peut citer à titre d'exemple la montmorillonite commercialisée sous la dénomination GEL WHITE H par la société ROCKWOOD ADDITIVES, la smectite purifiée commercialisée sous la dénomination VEEGUM GRANULES par la société VANDERBILT. On peut également citer la montmorillonite commercialisée sous la dénomination Kunipia G4 par la société Kunimine et la sépiolite Pangel S9 commercialisée par la société TOLSA.

On peut citer à titre d'exemples de kaolinites les kaolins commercialisés sous les dénominations COSLIN C 100 par la société BASF PERSONAL CARE INGREDIENTS ou KAOLIN SUPREME par la société IMERYS.

Les talcs sont des silicates de magnésium hydratés comprenant le plus souvent du silicate d'aluminium. La structure cristalline du talc consiste en des couches répétées d'un sandwich de brucite entre des couches de silice. A titre d'exemples, on peut citer le silicate de magnésium micronisé de granulométrie 5 microns commercialisé sous la dénomination MICRO ACE P3 par la société NIPPON TALC ou les talcs commercialisés sous les dénominations ROSE TALC et TALC SG-2000 par la société NIPPON TALC, , J 68 BC par la société US COSMETICS (MIYOSHI), LYZENAC 00 et LUZENAC PHARMA M par la société LUZENAC, et TALC JA-46R par la société ASADA MILLING.

A titre de saponite, qui appartient à la famille des montmorillonites, on peut citer la saponite synthétique, notamment celle vendue par la société Kunimine sous la dénomination SUMECTON®.

A titre d'exemple de laponite synthétique, on peut citer la laponite XLG commercialisée par la société Rockwood.

L'argile peut être présente dans la composition selon l'invention en une quantité allant de 0,1 à 50 % en poids, notamment de 1 à 30 % en poids et en particulier de 1 à 20 % en poids par rapport au poids total de la composition.

Les oxydes métalliques peuvent être choisis parmi le dioxyde de titane, les oxydes de fer, les oxydes de zirconium, les oxydes de zinc, les oxydes de cérium, les oxydes de chrome. On utilise de préférence les oxydes de fer ou le dioxyde de titane.

L'oxyde metallique peut être dans la composition selon l'invention en une quantité allant de 0,1 à 50 % en poids, notamment de 1 à 30 % en poids et en particulier de 1 à 20 % en poids par rapport au poids total de la composition.

La composition selon l'invention est généralement adaptée à une application topique sur les matières kératiniques, en particulier sur la peau, et comprend donc généralement un milieu physiologiquement acceptable, c'est-à-dire un milieu compatible avec la peau et/ou ses phanères. Il s'agit de préférence d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition.

La composition selon l'invention peut se présenter sous toutes les formes galéniques classiquement utilisées pour une application topique et notamment sous forme de dispersions du type lotion ou gel aqueux ou huileux, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou de dispersions cire/phase aqueuse. Ces compositions sont préparées selon les méthodes usuelles.

Selon un mode préféré de réalisation de l'invention, la composition se présente sous forme d'une émulsion H/E ou d'un gel huileux.

Avantageusement, la composition selon l'invention comprend une huile, notamment en une teneur pouvant aller de 50 à 99 % en poids.

La composition selon l'invention peut comprendre une huile volatile.

Par « huile volatile », on entend au sens de l'invention toute huile susceptible de s'évaporer au contact de la peau, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40.000 Pa (0,001 à 300 mm de Hg) et de préférence allant de 1,3 à 1300 Pa (0,01 à 10 mm de Hg).

Ces huiles volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permethyls®.

Comme huiles volatiles, on peut aussi utiliser les silicones volatiles, comme par exemple les huiles de silicones linéaires ou cycliques volatiles, notamment celles ayant une viscosité ≤ 5 centistokes ($5 \times 10^{-6}$ $m^2/s$), et ayant notamment de 2 à 10 atomes de silicium, de préférence de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane,

le décaméthyl tétrasiloxane, le dodécaméthyl pentasiloxane et leurs mélanges.

La composition selon l'invention peut comprendre une huile non volatile.

Par "huile non volatile", on entend une huile restant sur la peau à température ambiante et pression atmosphérique au moins plusieurs heures et ayant notamment une pression de vapeur inférieure à 1,33 Pa (0,01 mm de Hg).

Ces huiles non volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :

- les huiles hydrocarbonées d'origine animale,

- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810®, 812® et 818® par la société Dynamit Nobel,

- les éthers de synthèse ayant de 10 à 40 atomes de carbone ;

- les hydrocarbures linéaires ou ramifiés, d'origine minérale ou synthétique tels que la vaseline, les polydécènes, le polyisobutène hydrogéné tel que le Parleam ®, le squalane, les huiles de paraffine, et leurs mélanges,

- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit $\geq 10$, comme par exemple l'huile de Purcellin (octanoate de cétostéaryle), le myristate d'isopropyle, le palmitate d'isopropyle, les benzoates d'alcools en $C_{12}$ à $C_{15}$, le laurate d'hexyle, l'adipate de diisopropyle, l'isononanoate d'isononyle, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,

- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,

- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les triméthylsiloxyphenyl diméthicones, les phényl triméthylsiloxy diphénylsiloxanes, les diphényl diméthicones, les diphényl méthyldiphényl trisiloxanes, et leurs mélanges.

La composition peut être anhydre. On entend par composition anhydre une composition contenant moins de 2 % en poids d'eau, voire moins de 0,5 % d'eau, et notamment exempte d'eau. Le cas échéant, d'aussi faibles quantités d'eau peuvent notamment être amenées par des ingrédients de la composition qui peuvent en contenir des quantités résiduelles.

La composition selon l'invention peut contenir en outre contenir un ou plusieurs adjuvants couramment utilisés dans le domaine cosmétique, tels que des émulsionnants, des conservateurs, des séquestrants, des parfums, des épaississants, des huiles, des cires, des polymères filmogènes, des matières colorantes.

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 30 % en poids, par rapport au poids total de la composition.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène.

Bien entendu, l'homme du métier veillera à choisir ce ou ces éventuels composés additionnels et/ou leur quantité de manière telle que les propriétés anti-rides de la composition selon l'invention ne soient pas, ou substantiellement pas, altérées par l'adjonction envisagée.

Selon un premier mode de réalisation du procédé selon l'invention, on applique sur la peau un mélange extemporané d'une composition comprenant le polymère phosphonique et d'un composé aminé tel que décrits précédemment ou d'une composition le contenant et comprenant un milieu physiologiquement acceptable. Le mélange extemporané est avantageusement effectué moins de 5 minutes avant son application sur la peau, et de préférence moins de 3 minutes.

Selon un deuxième mode de réalisation du procédé selon l'invention, on applique sur la peau d'abord la composition comprenant le polymère phosphonique, puis on applique un composé aminé tel que décrit précédemment ou une composition le contenant et comprenant un milieu physiologiquement acceptable. L'application du composé aminé peut être effectuée après un temps compris entre 5 minutes et une heure après avoir appliqué le polymère phosphonique.

Selon un troisième mode de réalisation du procédé selon l'invention, on applique d'abord sur la peau le composé aminé, ou une composition le contenant et comprenant un milieu physiologiquement acceptable, puis on applique la composition cosmétique comprenant le polymère phosphonique. L'application du polymère phosphonique peut être effectuée après un temps compris entre 5 minutes et une heure après avoir appliqué le composé aminé.

L'application de la composition selon l'invention se fait selon les techniques habituelles, par exemple par application (notamment de crèmes, de gels, de sérums, de lotions) sur la peau destinée à être traitée, en particulier la peau du visage et/ou du cou, notamment la peau du contour de l'oeil. Dans le cadre de ce procédé, la composition peut être, par exemple, une composition de soin.

L'invention va maintenant être décrite en référence aux exemples suivants donnés à titre illustratif et non limitatif.

**Exemple 1** : Copolymère acrylate d'isobornyle / acide vinyl phosphonique (90/10 en poids)

Dans un réacteur, on a introduit 180 g d'acrylate d'isobornyle, 20 g d'acide vinyl phosphonique et 200 g d'un mélange isododécane / éthanol (70/30 poids/poids). Le milieu réactionnel a été dégazé sous argon pendant 20 minutes. Puis 2 g d'amorceur 2,5-diméthyl-2,5-di(2-éthylhexanoylperoxy)hexane (Trigonox® T141 de chez Akzo Nobel) ont été ajoutés. Le milieu réactionnel a été chauffé au reflux de l'éthanol pendant 24 heures sous agitation. Après refroidissement à température ambiante (25 °C) le milieu réactionnel a été dilué avec 300 g d'isododécane.

Le produit obtenu a été précipité dans l'éthanol, récupéré et séché à l'étuve 60°C sous vide.
On a obtenu après séchage 180 g (rendement 90 %) d'une poudre blanche.
Poids moléculaire Mw = 55700.
L'indice d'acide est de 21 mg/g.

**Exemple 2** : Copolymère acrylate d'isobornyle / acide vinyl phosphonique (80/20 en poids)

Le copolymère a été préparé selon le mode opératoire de l'exemple 1 en utilisant 160 g d'acrylate d'isobornyle et 40 g d'acide vinyl phosphonique.

On a obtenu 175 g (rendement 87,5 %) d'une poudre blanche.
Poids moléculaire MW = 67500 .
L'indice d'acide est de 31 mg/g.

**Exemple 3 (hors invention)** : Copolymère acrylate d'isobornyle / acide acrylique (90/10 en poids)

Le copolymère a été préparé selon le mode opératoire de l'exemple 1 en utilisant 180 g d'acrylate d'isobornyle et 20 g d'acide acrylique.

On a obtenu 140 g (rendement 70 %) d'une poudre blanche.
L'indice d'acide est de 18 mg/g.

**Exemple 4 (hors invention)** : Copolymère acrylate d'isobornyle / acide acrylique (80/20 en poids)

Le copolymère a été préparé selon le mode opératoire de l'exemple 1 en utilisant 160 g d'acrylate d'isobornyle et 40 g d'acide acrylique.

On a obtenu 150 g (rendement 75 %) d'une poudre blanche.
L'indice d'acide est de 110 mg/g.

**Exemple 5** :

**Mise en évidence de l'effet tenseur des polymères utilisés selon l'invention**

Cet essai consiste à comparer *in vitro* le pouvoir tenseur du polymère à évaluer par rapport à un polymère tenseur de référence : Hybridur® 875 polymer dispersion de chez Air Products (dispersion aqueuse à 40 % en poids de particules d'un réseau interpénétré de polymères polyuréthane et acrylique). Le polymère à évaluer est déposé sur une bandelette de caoutchouc nitrile découpée dans un gant vendu sous la référence « Safeskin Nitrile Criticial » n° 038846 par la société Dominique Dutscher SA, d'une surface de 3,5 $cm^2$ préalablement tendues sur un support. Une solution contenant le polymère à évaluer est donc déposée sur la bandelette d'élastomère, en déposant 1,8 mg (en matières sèches) de polymère.

On dépose ainsi sur une bandelette de caoutchouc nitrile 26 $\mu$l d'une solution aqueuse contenant 7 % MA de polymère Hybridur® 875 pour obtenir ainsi une bandelette de référence tenseur et sur une autre bandelette on dépose 26 $\mu$l d'une solution contenant 7 % MA de polymère phosphonique à évaluer dans un mélange isododécane/éthanol (70/30 poids/poids).

Après séchage pendant 24 heures à la température ambiante (25 °C), on observe le recourbement (rétractation) de la bandelette traitée avec le polymère phosphonique en comparaison avec celui obtenu avec le témoin (Hybridur® 875).

On a également évalué l'effet tenseur du polymère greffé en présence de 3-aminopropyltriéthoxysilane (APTES) ou de *O,O*'-Bis(2-aminopropyl) polypropylene glycol-*block*-polyethylene glycol-*block*-polypropylene glycol (Jeffamine® ED-600 de chez Huntsman) ou de PDMS diamine (DMS-A15 de chez Gelest).

Pour le 3-aminopropyltriéthoxysilane (APTES), on a préparé les mélanges suivants avant l'application sur la bandelette de nitrile (le polymère phosphonique étant en solution dans le mélange isododécane/éthanol) :

| Ex | Taux de neutralisation | Quantité de polymère de l'exemple 1 (en g) | Nombre moles d' acide phosphonique | Quantité d' APTES (en mg) | Nombre moles d'amines |
|---|---|---|---|---|---|
| 1a | 25% | 0,7 | $2{,}625.10^{-4}$ | 7,26 | $3{,}2813.\ 10^{-5}$ |
| 1b | 50% | 0,7 | $2{,}625.10^{-4}$ | 14,52 | $6{,}5625.\ 10^{-5}$ |
| 1c | 100% | 0,7 | $2{,}625.10^{-4}$ | 29,05 | $13{,}125.\ 10^{-5}$ |

| Ex | Taux de neutralisation | Quantité de polymère de l'exemple 2 (en g) | Nombre moles d' acide phosphonique | Quantité d' APTES (en mg) | Nombre moles d'amines |
|---|---|---|---|---|---|
| 2a | 25% | 0,7 | $4{,}125.\ 10^{-4}$ | 11,41 | $5{,}1563.10^{-5}$ |
| 2b | 50% | 0,7 | $4{,}125.\ 10^{-4}$ | 22,82 | $10{,}3125.\ 10^{-5}$ |
| 2c | 100% | 0,7 | $4{,}125.\ 10^{-4}$ | 45,64 | $20{,}625.\ 10^{-5}$ |

(suite)

| Ex | Taux de neutralisation | Quantité de polymère de l'exemple 2 (en g) | Nombre moles d' acide phosphonique | Quantité d' APTES (en mg) | Nombre moles d'amines |
|---|---|---|---|---|---|
| 2d | 200% | 0,7 | $4,125.\ 10^{-4}$ | 91,28 | $41,25.\ 10^{-5}$ |

Pour le *O,O'*-Bis(2-aminopropyl) polypropylene glycol-*block*-polyethylene glycol-*block*-polypropylene glycol (Jeffamine® ED-600 de chez Huntsman), on a préparé les mélanges suivants avant l'application sur la bandelette de nitrile (le polymère phosphonique étant en solution dans le mélange isododécane/éthanol) :

| ex | Taux de neutralisation | Quantité de polymère de l'exemple 1 (en g) | Nombre moles d'acide phosphonique | Quantité de Jeffamine® ED-600 (en mg) | Nombre moles d'amines |
|---|---|---|---|---|---|
| 1e | 25% | 0,7 | $2,625.10^{-4}$ | 10 | $3,2813.\ 10^{-5}$ |
| 1f | 100% | 0,7 | $2,625.\ 10^{-4}$ | 40 | $13,125.\ 10^{-5}$ |

Pour la PDMS diamine (DMS-A15 de chez Gelest), on a préparé les mélanges suivants avant l'application sur la bandelette de nitrile (le polymère phosphonique étant en solution dans le mélange isododécane/éthanol) :

| ex | Taux de neutralisation | Quantité de polymère de l'exemple 1 (en g) | Nombre moles d'acide phosphonique | Quantité PDMS diamine (en mg) | Nombre moles d'amines |
|---|---|---|---|---|---|
| 1g | 25% | 0,7 | $2,625.10^{-4}$ | 41 | $3,2813.\ 10^{-5}$ |
| 1h | 50% | 0,7 | $2,625.\ 10^{-4}$ | 82 | $6,5625.\ 10^{-5}$ |
| 1i | 100% | 0,7 | $2,625.\ 10^{-4}$ | 164 | $13,125.\ 10^{-5}$ |

Les mélanges préparés ont été déposés (26 µl) sur les bandelettes de caoutchouc nitrile.

On a mesuré l'effet tenseur obtenu selon le protocole décrit précédemment.

Puis on a évalué la résistance à l'eau de l'effet tenseur en plongeant les bandelettes de caoutchouc traitées avec le polymère à évaluer dans l'eau à la température ambiante (25 °C) pendant 10 minutes.

On a obtenu les résultats suivants :

| **Polymère testé** | **Effet tenseur** | **Effet tenseur après immersion dans l'eau** |
|---|---|---|
| Référence Hybridure 875 | correct | correct |
| Exemple 1 | idem à la référence | Idem à la référence |
| Exemple 1a | supérieur à la référence | supérieur à la référence |
| Exemple 1b | Idem à la référence | idem à la référence |
| Exemple 1c | idem à la référence | idem à la référence |
| Exemple 2 | idem à la référence | idem à la référence |
| Exemple 2a | idem à la référence | supérieur à la référence |
| Exemple 2b | idem à la référence | supérieur à la référence |
| Exemple 2c | supérieur à la référence | supérieur à la référence |
| Exemple 2d | supérieur à la référence | supérieur à la référence |
| Exemple 1e | inférieur à la référence mais effet tenseur observé | inférieur à la référence mais effet tenseur observé |

(suite)

| Polymère testé | Effet tenseur | Effet tenseur après immersion dans l'eau |
|---|---|---|
| Exemple 1f | inférieur à la référence mais effet tenseur observé | inférieur à la référence mais effet tenseur observé |
| Exemple 1g | inférieur à la référence mais effet tenseur observé | idem à la référence |
| Exemple 1h | idem à la référence | idem à la référence |
| Exemple 1i | Idem à la référence | idem à la référence |

Les résultats obtenus montrent que les polymères phosphonique des exemples 1 et 2 seuls ou mélangés avec les composés aminés ont un bon effet tenseur, même après immersion dans l'eau : l'effet tenseur est rémanent à l'eau.

**Exemple 6 (comparatif)** :

On a évalué la qualité des films obtenus avec les polymères des exemples 1 et 2 selon l'invention et aussi avec les polymères des exemples 3 et 4 hors invention.
Les polymères ont été mis en solution à 7 % MA dans un mélange isododécane/éthanol (70/30). On a appliqué 26 µL de la solution de polymère sur une bande de caoutchouc nitrile (telle que décrite dans l'exemple précédent) et on a observé l'aspect du film obtenu après séchage à l'air pendant 24 heures à la température ambiante .
Les polymères 1 et 2 selon l'invention forment des films transparents et homogènes, tandis que les polymères 3 et 4 hors invention forment des films blancs (non transparents) et non homogènes (film effrité).

**Exemple 7** :

On prépare un gel anti-rides ayant la composition suivante :

- polymère de l'exemple 1 7 g
- disteardimonium hectorite/ppropylene carbonate dans isodoecane (bentone gel ® ISDV de chez Elementis) 3 g
- Conservateurs qs
- Isododecane/éthanol (80/20 p/p) qsp 100 g

Une composition similaire a également été préparée en utilisant le polymère de l'exemple 2.
La composition obtenue appliquée sur le visage permet de lisser efficacement les rides.

**Exemple 8** :

On prépare un gel anti-rides ayant la composition suivante :

- polymère de l'exemple 1 7 g
- disteardimonium hectorite/ppropylene carbonate dans isodoecane (bentone gel ® ISDV de chez Elementis) 3 g
- Conservateurs qs
- Isododecane/éthanol (80/20 p/p) qsp 100 g

Juste avant l'application sur la peau, on ajoute dans le gel 145,2 mg de 3-aminopropyltriethoxysilane (APTES).
La composition obtenue, en mélange avec l'APTES, appliquée sur le visage permet de lisser efficacement les rides.

**Exemple 9 :**

On prépare un gel anti-rides ayant la composition suivante :

- polymère de l'exemple 2 7 g
- disteardimonium hectorite/ppropylene carbonate dans isodoecane (bentone gel ® ISDV de chez Elementis) 3 g
- Conservateurs qs
- Isododecane/éthanol (80/20 p/p) qsp 100 g

Juste avant l'application sur la peau, on ajoute dans le gel 228,2 mg de 3-aminopropyltriethoxysilane (APTES).
La composition obtenue, en mélange avec l'APTES, appliquée sur le visage permet de lisser efficacement les rides.

**Exemple 10 :**

On prépare un gel anti-rides ayant la composition suivante :

- polymère de l'exemple 1 7 g
- disteardimonium hectorite/ppropylene carbonate dans isodoecane (bentone gel ® ISDV de chez Elementis) 3 g
- Conservateurs qs
- Isododecane/éthanol (80/20 p/p) qsp 100 g

Juste avant l'application sur la peau, on ajoute dans le gel 820 mg de PDMS diamine (DMS-A15 de chez Gelest) .
La composition obtenue, en mélange avec la PDMS diamine, appliquée sur le visage permet de lisser efficacement les rides.

**Exemple 11 :**

On prépare un gel anti-rides ayant la composition suivante :

- polymère de l'exemple 2 7 g
- disteardimonium hectorite/ppropylene carbonate dans isodoecane (bentone gel ® ISDV de chez Elementis) 3 g
- Conservateurs qs
- Isododecane/éthanol (80/20 p/p) qsp 100 g

On applique la composition sur la peau ridée du visage. On laisse sécher pendant 1 heure.
Puis on applique sur la zone de la peau traitée la solution suivante :

- 3-aminopropyltriethoxysilane (APTES) 145,2 mg
- Isododecane/éthanol (80/20 p/p) qsp 100 g

et on laisse sécher 1 heure.
L'application séquentielle des 2 compositions sur le visage permet de lisser efficacement les rides.

**Exemples 12 à 14 :**

On a préparé les 3 compositions décrites ci-dessous.
On a appliqué chaque composition sur un support équivalent de peau en élastomère en réalisant un dépôt d'une épaisseur de 100 µm humide et on a laissé sécher à température ambiante (25 °C) pendant 24 heures.
On a ensuite observé l'état du film obtenu.
On a évalué la résistance du film obtenu en appliquant séparément 0,5 ml d'huile d'olive et 0,5 ml de sébum ; après 5 minutes de contact on a frotté la surface du film avec un coton puis on a observé l'état du film .
On a également évalué l'aspect collant du film et son aptitude à transférer ou non en touchant le film avec le doigt.
On a obtenu les résultats suivants :

| | Exemple 12 | Exemple 13 | Exemple 14 |
|---|---|---|---|
| Polymère de l'exemple 1 | 25 g | 25 g | 25 g |
| PDMS diamine (DMS-A15 de chez Gelest) | 0 | 3,75 g | 7,5 g |
| Isododécane:éthano l (70/30 p/p) | qsp 100 g | qsp 100 g | qsp 100 g |
| Aspect du film | Film homogène | Film homogène | Film homogène |
| Résistance à l'huile d'olive | + | +++ | +++ |
| Résistance au sébum | + | +++ | +++ |
| Non collant | +++ | +++ | +++ |
| Non transfert | +++ | +++ | +++ |

Les résultats obtenus montrent que le polymère 1 seul ou en présence de la PDMS diamine forme un film homogène non collant et ne transférant pas au doigt. La résistance du film au contact de l'huile d'olive et du sébum est nettement améliorée en présence de la PDMS diamine.

**Exemples 15 à 18 :**

On a préparé les 4 compositions décrites ci-dessous.
On a ensuite effectué l'évaluation des propriétés filmogène selon les protocoles décrits dans les exemples 12 à 14.
On a obtenu les résultats suivants :

| | Ex15 | Ex 16 | Ex 17 | Ex 18 |
|---|---|---|---|---|
| Polymère de l'exemple 1 | 25 g | 25 g | 25 g | 25 g |
| PDMS diamine (DMS-A15 de chez Gelest) | 0 | 3,75 | | 3,75 g |
| pâte pigmentaire 40 % en poids d'oxyde de fer dans l'isododécane | - | - | 5 g | 5 g |
| Octyl-2 dodécanol | 20 g | 20 | 20 | 20 g |
| Isododécane | qsp 100 g | qsp 100 g | qsp 100 g | qsp 100 g |
| Aspect du film | Film homogène | Film homogène | Film homogène | Film homogène |
| Résistance à l'huile d'olive | + | +++ | +++ | +++ |
| Résistance au sébum | + | +++ | +++ | +++ |
| Non collant | + | +++ | + | +++ |
| Non transfert | +++ | +++ | +++ | +++ |

Les résultats obtenus montrent que le polymère 1 seul (ex 15) ou en présence de la PDMS diamine (ex 16) et formulé avec l'octyl-2 dodécanol (huile non volatile) forme un film homogène non collant et ne transférant pas au doigt. La résistance du film au contact de l'huile d'olive et du sébum est nettement améliorée en présence de la PDMS diamine.
Lorsque l'on ajoute de l'oxyde de fer, on constate une amélioration de la résistance à l'huile d'olive et au sébum (exemples 17 et 18 en comparaison avec l'exemple 15).

**Exemples 19:**

On a préparé la composition de fond de teint décrite ci-dessous.
On a ensuite effectué l'évaluation des propriétés filmogène selon les protocoles décrits dans les exemples 12 à 14.
On a obtenu les résultats suivants :

| | Exemple 19 |
|---|---|
| Polymère de l'exemple 1 | 25 g |
| pâte pigmentaire 40 % en poids d'oxyde de fer dans l'isododécane | 5 g |
| Disteardimonium Hectorite (BENTONE GEL ISD V de chez Elementis) | 10 g |
| Octyl-2 dodécanol | 20 g |
| Isododécane : | qsp 100 g |
| Aspect du film | Film homogène |
| Résistance à l'huile d'olive | +++ |
| Résistance au sébum | +++ |
| Non collant | +++ |
| Non transfert | +++ |

Les résultats obtenus montrent que la composition de fond de teint forme un film homogène non collant et ne transférant pas au doigt. Le film obtenu présente également une bonne résistance au contact de l'huile d'olive et du sébum, nettement améliorée en présence de la bentone par rapport au polymère seul (exemple 15).

Le fond de teint appliqué sur la peau du visage permet ainsi d'obtenir un maquillage non collant, non transfert et résistant au sébum donc présentant une bonne tenue.

**Revendications**

1. Copolymère issu de la polymérisation de :

   (i) 70 à 95 % en poids, du poids total des monomères, de (méth)acrylate d'isobornyle ;
   (ii) 1 à 30 % en poids de monomère acide vinyl phosphonique de formule (I) :

   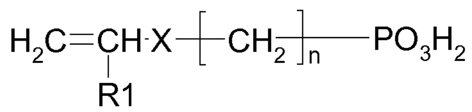

   (I)

   dans laquelle :

   R1 désigne H ou $-CH_3$ ;
   X désigne une liaison covalente et n désigne un nombre entier allant de 0 à 14 ;
   ou X désigne un groupement -COO- et n désigne un nombre entier allant de 2 à 6 ;

   (iii) 0 à 20 % en poids d'un monomère additionnel, qui est différent des monomères de (méth)acrylate d'isobornyle et d'acide vinyl phosphonique.

2. Copolymère selon la revendication 1, **caractérisé par le fait que** pour le monomère (I) :
   X désigne une liaison covalente et n est un nombre entier allant de 0 à 6 ou X désigne un groupe -COO- et n est un nombre entier allant de 2 à 4.

3. Copolymère selon l'une des revendications précédentes, **caractérisé par le fait que** pour le monomère (I), R1 = H et X désigne une liaison covalente et n est un nombre entier allant de 0 à 4.

4. Copolymère selon l'une des revendications précédentes, **caractérisé par le fait que** le monomère (I) est choisi parmi :

   l'acide vinyl phosphonique ;

l'acide 3-butenyl phosphonique ;
l'acide 4-pentenyl phosphonique ;
l'acide 10-undecenyl phosphonique ;
l'acide 11-dodecenyl phosphonique ;
l'ester 2-phosphonoethyl de l'acide 2-methyl 2- propenoïque ;
l'ester 2-phosphonoethyl de l'acide 2- propenoïque ;
et de préférence l'acide vinyl phosphonique.

**5.** Copolymère selon l'une des revendications précédentes, **caractérisé par le fait que** le monomère additionnel est un monomère de formule (II) :

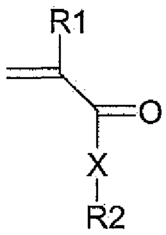

(II)

dans laquelle :

$R_1$ désigne un atome d'hydrogène ou un radical méthyle ;
X désigne O ou NH ou $NR_3$ ;
$R_2$ désigne ou radical alkyl linéaire en $C_1$-$C_{22}$ ou ramifié en $C_3$-$C_{10}$ ou cyclique en $C_5$-$C_7$ ou un radical hydrocarboné insaturé linéaire en $C_3$-$C_{20}$ ou ramifié en $C_6$-$C_{20}$ ou cyclique en $C_5$-$C_7$ ou un radical $-(Si(CH_3)_2O)_b-CH_3$ avec b allant de 5 à 70, étant entendu que X = O lorsque $R_2$ est un radical de formule $-(Si(CH_3)_2O)_b-CH_3$ ;
$R_3$ désigne un radical alkyle linéaire en $C_1$-$C_{12}$ ou ramifié en $C_3$-$C_{12}$.

**6.** Copolymère selon l'une des revendications précédentes, **caractérisé par le fait qu'**il est issu de la polymérisation de :

- 70 à 95 % en poids, du poids total des monomères, de (méth)acrylate d'isobornyle ;
- 5 à 30 % en poids d'acide vinyl phosphonique ;

et de préférence de :

- 75 à 95 % en poids, du poids total des monomères, de (méth)acrylate d'isobornyle ;
- 5 à 25 % en poids d'acide vinyl phosphonique.

**7.** Copolymère selon l'une quelconque des revendications précédentes, **caractérisé par le fait qu'**il est choisi parmi les copolymères acrylate d'isobornyle / acide vinylphosphonique (80/20) ou 90/10 (poids/poids).

**8.** Composition comprenant, dans un milieu physiologiquement acceptable, un copolymère selon l'une quelconque des revendications précédentes.

**9.** Composition selon la revendication précédente, **caractérisée par le fait que** le copolymère est présent en une teneur allant de 0,1 à 10 % en poids, par rapport au poids total de la composition, de préférence de 0,5 % à 10 % en poids de matière active, et préférentiellement allant de 1 % à 8 % en poids, et plus préférentiellement allant de 1 % à 6 % en poids.

**10.** Composition selon l'une des revendications 8 ou 9, **caractérisée par le fait qu'**elle comprend une matière colorante.

**11.** Composition selon la revendication précédente, **caractérisée par le fait que** la matière colorante est choisie parmi les pigments.

**12.** Procédé de soin ou de maquillage des matières kératiniques comprenant l'application topique sur les matières

kératiniques d'une composition selon l'une des revendications 8 à 11.

**13.** Procédé de maquillage des matières kératiniques comprenant l'application topique sur les matières kératiniques d'une composition selon l'une des revendications 10 ou 11.

**14.** Procédé selon la revendication 12 ou 13, **caractérisé par le fait que** l'on effectue l'application topique sur les matières kératiniques d'un mélange extemporané de la composition selon l'une des revendications 8 à 11 et d'un composant additionnel choisi parmi :

(i) un composé aminé choisi parmi les composés aminés ayant plusieurs groupes amine primaire et/ou amine secondaire et les aminosilanes,
(ii) les sels d'ions de métal divalent ou trivalent,
(iii) les argiles
(iv) les oxydes metalliques

ou d'une composition le contenant et comprenant un milieu physiologiquement acceptable.

**15.** Procédé selon l'une des revendications 12 ou 13, **caractérisé par le fait que** l'on effectue l'application séquentielle sur les matières kératiniques de la composition selon l'une des revendications 8 à 11 et d'un composant additionnel choisi parmi :

(i) un composé aminé choisi parmi les composés aminés comprenant plusieurs groupes amine primaire et/ou amine secondaire et les aminosilanes,
(ii) (ii) les sels d'ions de métal divalent ou trivalent,
(iii) les argiles
(iv) les oxydes metalliques

ou d'une composition le contenant et comprenant un milieu physiologiquement acceptable.

**16.** Procédé selon l'une quelconque des revendications 14 ou 15, **caractérisé par le fait que** le composé aminé comprend de 2 à 20 atomes de carbone.

**17.** Procédé selon l'une quelconque des revendications 14 à 16, **caractérisé par le fait que** le composé aminé est choisi parmi le N-méthyl-1,3-diaminopropane, le N-propyl 1,3-diaminopropane, le N-isopropyl 1,3-diaminopropane, le N-cyclohexyl 1,3-diaminopropane, le 2-(3-aminopropylamino) éthanol, le 3-(2-aminoéthyl)aminopropylamine, le bis(3-aminopropyl)amine, la méthyl bis(3-aminopropyl)amine, le N-(3-aminopropyl)-1,4-diaminobutane, la N,N-diméthyldipropylène triamine, le 1,2-bis(3-aminopropylamino)éthane, la N,N'-bis(3-aminopropyl)-1,3-propanediamine, l'éthylène diamine, la 1,3-propylènediamine, la 1,4-butylènediamine, la lysine, la cystamine, la xylène diamine, la tris(2-aminoéthyl)amine, la spermidine ;
les aminosilanes de formule (III) :

$$R'_1Si(OR'_2)_z(R'_3)_x \qquad (III)$$

dans laquelle :

• $R'_1$ est une chaîne hydrocarbonée en $C_1$-$C_6$, linéaire ou ramifiée, saturée ou insaturée, cyclique ou acyclique substituée par un groupement choisi parmi les groupements :

- amine $NH_2$ ou NHR avec R= alkyle en $C_1$-$C_4$,
- un groupement aryle ou aryloxy substitué par un groupement amino ou par un groupement aminoalkyl en $C_1$-$C_4$;

$R'_1$ pouvant être interrompu dans sa chaine par un hétéroatome (O, S, NH) ou un groupement carbonyle (CO), $R'_1$ étant lié à l'atome de silicium directement via un atome de carbone,

• $R'_2$ et $R'_3$ identiques ou différents, représentent un groupe alkyle, linéaire ou ramifié, comprenant de 1 à 6 atomes de carbone,
• z désigne un nombre entier allant de 1 à 3, et
• x désigne un nombre entier allant de 0 à 2,

avec z+x=3 ;
et de préférence choisi parmi l'éthylène diamine, la lysine, le 3-aminopropyltriethoxy-silane, et de préférence est le 3-aminopropyltriethoxysilane.

**18.** Procédé selon l'une des revendications 14 ou 15, **caractérisé par le fait que** le composé aminé est choisi parmi les polymères aminés, notamment ayant un poids moléculaire moyen en poids, determiné par chromatographie par exclusion stérique, allant de 500 à 1 000 000, de préférence allant de 500 à 500 000, et préférentiellement allant de 500 à 100 000.

**19.** Procédé selon la revendication précédente, **caractérisé par le fait que** le composé aminé est un polymère aminé choisi parmi les poly(alkylène ($C_2$-$C_5$) imines), et en particulier les polyéthylèneimines et les polypropylèneimines, notamment les poly(éthylène imine); la poly(allylamine) ; les polyvinylamines et leurs copolymères notamment avec des vinylamides; les copolymères vinylamine/vinylformamide ; les polyacides aminés présentant des groupes $NH_2$ comme la polylysine ; l'amino dextrane,; l'amino alcool polyvinylique, les copolymères à base d'acrylamidopropylamine; les chitosanes ;
les polydiméthylsiloxanes comprenant des groupes amines primaires en bout de chaine ou sur des chaines latérales, par exemple des groupes terminaux ou latéraux aminopropyl, comme par exemple ceux de formule (A) ou (B) ou (C) :

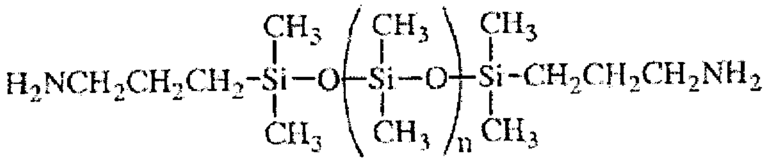

(A)

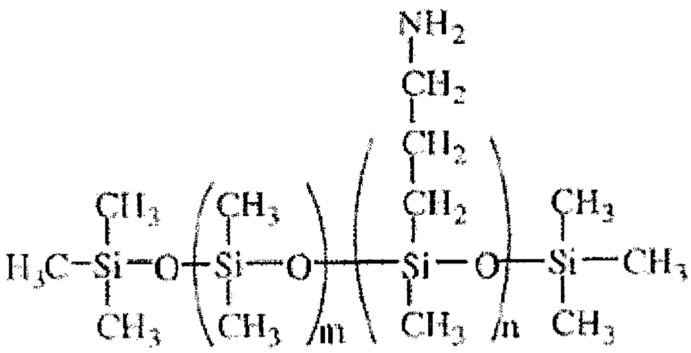

(B)

$$H_2NCH_2CH_2CH_2\text{-}Si(CH_3)_2\text{-}O\text{-}[Si(CH_3)_2\text{-}O]_n\text{-}Si(CH_3)_2C_4H_9 \quad (C)$$

avec :

dans la formule (A) : la valeur de n est telle que le poids moléculaire moyen en poids de la silicone est compris entre 500 et 55 000.
dans la formule (B), les valeurs de n et m sont telles que le poids moléculaire moyen en poids de la silicone est compris entre 1000 et 55 000.
dans la formule (C), la valeur de n est telle que le poids moléculaire moyen en poids de la silicone est compris entre 500 et 3000 ;

les amodiméthicones de formule (D) :

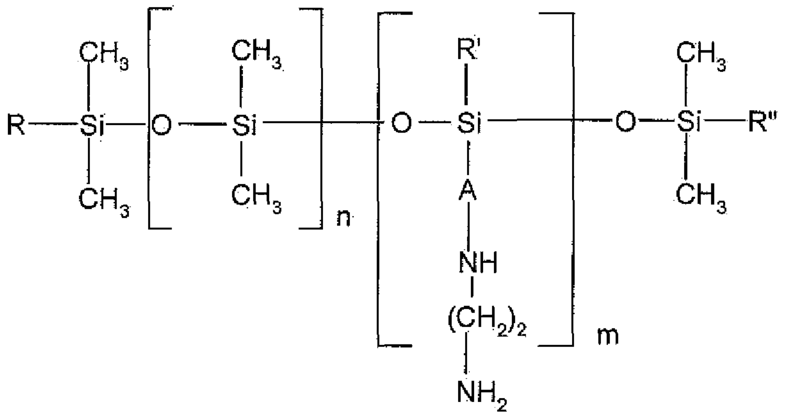

(D)

dans laquelle R, R' et R", identiques ou différents, représentent chacun un groupe alkyle en $C_1$-$C_4$ ou hydroxyle, A représente un groupe alkylène en $C_3$ et m et n sont tels que la masse moléculaire moyenne en poids du composé est comprise entre 5 000 et 500 000 environ.
Les polyéthers diamines et notamment les polyéthylèneglycol et/ou polypropylèneglycol $\alpha$, $\omega$-diamine ; les polytétrahydrofurane (ou polytétraméthylèneglycol) $\alpha$, $\omega$-diamine, les polybutadiènes $\alpha$, $\omega$-diamine,
Les dendrimères polyamidoamine à fonctions amines terminales,
Les poly(méth) acrylates ou poly(méth)acrylamides porteurs de fonctions amines primaires ou secondaires latérales telles que le poly(3-aminopropyl)méthacrylamide, le poly(2-aminoéthyl) méthacrylate ;
et de préférence les polyéthylèneglycol et/ou polypropylèneglycol $\alpha$, $\omega$-diamine et les polydiméthyisiloxanes comprenant des groupes terminaux aminopropyl.

**20.** Procédé selon l'une quelconque des revendications 14 à 19, **caractérisé par le fait que** le composé aminé est mis en oeuvre selon un ratio molaire composé aminé / acide phosphonique allant de 0,01 à 10, de préférence allant de 0,1 à 5, préférentiellement allant de 0,1 à 2, et plus préférentiellement allant de 0,1 à 1.

**21.** Procédé selon l'une quelconque des revendications 14 et 15, **caractérisé par le fait que** le composant additionnel est une argile choisie parmi les argiles de la famille des smectites telles que la laponite et la montmorillonite, de la famille de la kaolinite telles que la kaolinite, la dickite, la nacrite, les argiles éventuellement modifiées de la famille de l'halloysite, de la dombassite, de l'antigorite, de la benthiérine, de la pyrophyllite, des montmorillonites, de la beidellite, des vermiculites, du talc, de la stévensite, des hectorites, des bentonites, des saponites, des chlorites, de la sépiolite et de l'illite ; et de préférence choisies parmi la kaolinite, les smectites telles que la laponite et la montmorillonite, la bentonite et la saponite.

**22.** Procédé selon l'une quelconque des revendications 14 et 15, **caractérisé par le fait que** le composant additionnel est un sel d'ions de métal divalent ou trivalent choisis parmi les sels d'ions issus de Al(III), Ca(II), Cu(II), Fe(II), Fe(III), Mg(II), Mn(II), Zn(II) et leurs mélanges.

**23.** Procédé selon l'une quelconque des revendications 14 et 15, **caractérisé par le fait que** le composant additionnel est un oxyde métalliques choisi parmi le dioxyde de titane, les oxydes de fer, les oxydes de zirconium, les oxydes de zinc, les oxydes de cérium, les oxydes de chrome.

**24.** Procédé selon l'une quelconque des revendications 14, 17 à 23, **caractérisé par le fait que** l'on applique sur les matières kératiniques, notamment sur la peau, un mélange extemporané effectué moins de 5 minutes avant l'application sur les matières kératiniques, notamment sur la peau, de la composition selon l'une des revendication 8 à 11 et dudit composant additionnel , ou d'une composition le contenant et comprenant un milieu physiologiquement acceptable.

**25.** Procédé selon l'une quelconque des revendications 15 à 24, **caractérisé par le fait que** l'on applique sur les matières kératiniques, en particulier sur la peau, d'abord la composition selon l'une des revendications 8 à 11 puis on applique ledit composant additionnel ou une composition le contenant et comprenant un milieu physiologiquement acceptable.

**26.** Procédé selon l'une quelconque des revendications 15 à 24, **caractérisé par le fait que** l'on applique sur les

matières kératiniques, en particulier sur la peau d'abord ledit composant additionnel, ou une composition le contenant et comprenant un milieu physiologiquement acceptable, puis on applique la composition selon la revendication 8 ou 9.

**27.** Procédé selon l'une quelconque des revendications 12 à 26, **caractérisé en ce que** la composition comprend une huile.

**28.** Procédé selon l'une quelconque des revendications 12 à 27, destiné au soin de la peau, plus particulièrement de la peau du visage, en particulier de la peau ridée.

**29.** Procédé selon l'une quelconque des revendications 12 à 27, **caractérisé en ce qu'**il est destiné à atténuer les rides.

**30.** Utilisation cosmétique en tant qu'agent tenseur de la peau, en particulier d'une peau ridée, d'un polymère phosphonique tel que défini dans l'une quelconque des revendications 1 à 7 et éventuellement en mélange avec un composé additionnel tel que défini dans l'une des revendications 14 à 19 et 21 à 23.

**31.** Composition obtenue par mélange d'une composition selon l'une des revendications 8 à 11 et d'un composant additionnel tel que défini dans l'une des revendications 14 à 19 et 21 à 23, ou d'une composition le contenant et comprenant un milieu physiologiquement acceptable.

**32.** Kit comprenant une première composition selon l'une des revendications 8 à 11 et une deuxième composition comprenant un composé additionnel tel que défini dans l'une des revendications 14 à 19 et 21 à 23 et comprenant un milieu physiologiquement acceptable, les première et deuxième compositions étant conditionnées chacune dans un ensemble de conditionnement distinct

## Patentansprüche

**1.** Copolymer aus der Polymerisation von:

(i) 70 bis 95 Gew.-%, bezogen auf das Gesamtgewicht der Monomere, Isobornyl(meth)acrylat;
(ii) 1 bis 30 Gew.-% Vinylphosphonsäure-Monomer der Formel (I) :

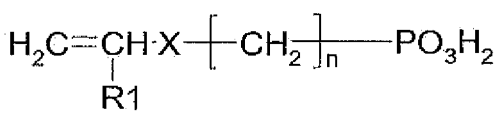

(I)

in der:

R1 für H oder -$CH_3$ steht;
X für eine kovalente Bindung steht und n für eine ganze Zahl im Bereich von 0 bis 14 steht oder X für eine -COO-Gruppe steht und n für eine ganze Zahl im Bereich von 2 bis 6 steht;

(iii) 0 bis 20 Gew.-% eines zusätzlichen Monomers, das von den Isobornyl(meth)acrylat- und Vinylphosphonsäure-Monomeren verschieden ist.

**2.** Copolymer nach Anspruch 1, **dadurch gekennzeichnet, dass** für das Monomer (I):
X für eine kovalente Bindung steht und n eine ganze Zahl im Bereich von 0 bis 6 ist oder X für eine -COO-Gruppe steht und n eine ganze Zahl im Bereich von 2 bis 4 ist.

**3.** Copolymer nach einem der vorhergehenden Ansprüche, **dadurch gekennzeichnet, dass** für das Monomer (I) R1 = H und X für eine kovalente Bindung steht und n eine ganze Zahl im Bereich von 0 bis 4 ist.

**4.** Copolymer nach einem der vorhergehenden Ansprüche, **dadurch gekennzeichnet, dass** das Monomer (I) aus:

Vinylphosphonsäure;
3-Butenylphosphonsäure;
4-Pentenylphosphonsäure ;

10-Undecenylphosphonsäure;
11-Dodecenylphosphonsäure;
2-Methyl-2-propensäure-2-phosphonoethylester;
2-Propensäure-2-phosphonoethylester
und bevorzugt Vinylphosphonsäure
ausgewählt ist.

**5.** Copolymer nach einem der vorhergehenden Ansprüche, **dadurch gekennzeichnet, dass** es sich bei dem zusätzlichen Monomer um ein Monomer der Formel (II):

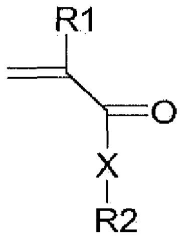

(II)

handelt, in der

$R_1$ für ein Wasserstoffatom oder einen Methylrest steht;
X für O oder NH oder $NR_3$ steht;
$R_2$ für einen linearen $C_1$-$C_{22}$-Alkylrest oder verzweigten $C_3$-$C_{10}$-Alkylrest oder cyclischen $C_5$-$C_7$-Alkylrest oder einen ungesättigten linearen $C_3$-$C_{20}$-Kohlenwasserstoffrest oder verzweigten $C_6$-$C_{20}$-Kohlenwasserstoffrest oder cyclischen $C_5$-$C_7$-Kohlenwasserstoffrest oder einen $-(Si(CH_3)_2O)_b-CH_3$-Rest steht, wobei b im Bereich von 5 bis 70 liegt, mit der Maßgabe, dass X = O, wenn $R_2$ ein Rest der Formel $-(Si(CH_3)_2O)_b-CH_3$ ist ;
$R_3$ für einen linearen $C_1$-$C_{12}$-Alkylrest oder verzweigten $C_3$-$C_{12}$-Alkylrest steht.

**6.** Copolymer nach einem der vorhergehenden Ansprüche, **dadurch gekennzeichnet, dass** es sich aus der Polymerisation von:

- 70 bis 95 Gew.-%, bezogen auf das Gesamtgewicht der Monomere, Isobornyl(meth)acrylat und
- 5 bis 30 Gew.-% Vinylphosphonsäure

und bevorzugt aus:

- 75 bis 95 Gew.-%, bezogen auf das Gesamtgewicht der Monomere, Isobornyl(meth)acrylat und
- 5 bis 25 Gew.-% Vinylphosphonsäure

ergibt.

**7.** Copolymer nach einem der vorhergehenden Ansprüche, **dadurch gekennzeichnet, dass** es aus Copolymeren von Isobornylacrylat und Vinylphosphonsäure im Gewichtsverhältnis 80/20 oder 90/10 ausgewählt ist.

**8.** Zusammensetzung, die in einem physiologisch unbedenklichen Medium ein Copolymer nach einem der vorhergehenden Ansprüche umfasst.

**9.** Zusammensetzung nach dem vorhergehenden Anspruch, **dadurch gekennzeichnet, dass** das Copolymer in einem Gehalt im Bereich von 0,1 bis 10 Gew.-%, bezogen auf das Gesamtgewicht der Zusammensetzung, vorzugsweise von 0,5 bis 10 Gew.-% Wirkstoff und bevorzugt im Bereich von 1 bis 8 Gew. -% und weiter bevorzugt im Bereich von 1 bis 6 Gew.-% vorliegt.

**10.** Zusammensetzung nach einem der Ansprüche 8 oder 9, **dadurch gekennzeichnet, dass** sie ein Farbmittel umfasst.

**11.** Zusammensetzung nach dem vorhergehenden Anspruch, **dadurch gekennzeichnet, dass** das Farbmittel aus Pigmenten ausgewählt ist.

**12.** Verfahren zur Pflege oder zum Schminken von Keratinmaterialien, umfassend die topische Applikation einer Zusammensetzung nach einem der Ansprüche 8 bis 11 auf die Keratinmaterialien.

**13.** Verfahren zum Schminken von Keratinmaterialien, umfassend die topische Applikation einer Zusammensetzung nach einem der Ansprüche 10 order 11 auf die Keratinmaterialien.

**14.** Verfahren nach Anspruch 12 oder 13, **dadurch gekennzeichnet, dass** man die topische Applikation einer extemporalen Mischung der Zusammensetzung nach einem der Ansprüche 8 bis 11 und einer zusätzlichen Komponente, die aus:

(i) einer Aminverbindung, die aus Aminverbindungen mit mehreren primären Amingruppen und/oder sekundären Amingruppen und Aminosilanen ausgewählt ist,
(ii) Salzen von zweiwertigen oder dreiwertigen Metallionen,
(iii) Tonen,
(iv) Metalloxiden

ausgewählt ist, oder einer Zusammensetzung, die diese enthält und ein physiologisch unbedenkliches Medium umfasst, auf die Keratinmaterialien durchführt.

**15.** Verfahren nach einem der Ansprüche 12 oder 13, **dadurch gekennzeichnet, dass** man die aufeinanderfolgende Applikation der Zusammensetzung nach einem der Ansprüche 8 bis 11 und einer zusätzlichen Komponente, die aus:

(i) einer Aminverbindung, die aus Aminverbindungen mit mehreren primären Amingruppen und/oder sekundären Amingruppen und Aminosilanen ausgewählt ist,
(ii) Salzen von zweiwertigen oder dreiwertigen Metallionen,
(iii) Tonen,
(iv) Metalloxiden

ausgewählt ist, oder einer Zusammensetzung, die diese enthält und ein physiologisch unbedenkliches Medium umfasst, auf die Keratinmaterialien durchführt.

**16.** Verfahren nach einem der Ansprüche 14 oder 15, **dadurch gekennzeichnet, dass** die Aminverbindung 2 bis 20 Kohlenstoffatome umfasst.

**17.** Verfahren nach einem der Ansprüche 14 bis 16, **dadurch gekennzeichnet, dass** die Aminverbindung aus N-Methyl-1,3-diaminopropan, N-Propyl-1,3-diaminopropan, N-Isopropyl-1,3-diaminopropan, N-Cyclohexyl-1,3-diaminopropan, 2-(3-Aminopropylamino)ethanol, 3-(2-Aminoethyl)aminopropylamin, Bis(3-aminopropyl)amin, Methylbis(3-aminopropyl)amin, N-(3-Aminopropyl)-1,4-diaminobutan, N,N-Dimethyldipropylentriamin, 1,2-Bis(3-aminopropylamino)ethan, N,N'-Bis(3-aminopropyl)-1,3-propandiamin, Ethylendiamin, 1,3-Propylendiamin, 1,4-Butylendiamin, Lysin, Cystamin, Xyloldiamin, Tris(2-aminoethyl)amin und Spermidin;
den Aminosilanen der Formel (III):

$$R'_1Si(OR'_2)_z(R'_3)_x \qquad (III)$$

in der:

• $R'_1$ eine lineare oder verzweigte, gesättigte oder ungesättigte, cyclische oder acyclische $C_1$-$C_6$-Kohlenwasserstoffkette ist, die durch eine Gruppe substituiert ist, die aus den folgenden Gruppen ausgewählt ist:

- Amin $NH_2$ oder NHR mit R = $C_1$-$C_4$-Alkyl,
- einer Aryl- oder Aryloxygruppe, die durch eine Aminogruppe oder durch eine $C_1$-$C_4$-Aminoalkylgruppe substituiert ist,

wobei $R'_1$ in seiner Kette durch ein Heteroatom (O, S, NH) oder eine Carbonylgruppe (CO) unterbrochen sein kann, wobei $R'_1$ über ein Kohlenstoffatom direkt an das Siliciumatom gebunden ist,
• $R'_2$ und $R'_3$ gleich oder verschieden sind und für eine lineare oder verzweigte Alkylgruppe mit 1 bis 6 Kohlenstoffatomen stehen,
• z für eine ganze Zahl im Bereich von 1 bis 3 steht und

- x für eine ganze Zahl im Bereich von 0 bis 2 steht,

wobei

$$z + x = 3;$$

ausgewählt ist und bevorzugt aus Ethylendiamin, Lysin und 3-Aminopropyltriethoxysilan ausgewählt ist und bevorzugt 3-Aminopropyltriethoxysilan ist.

**18.** Verfahren nach einem der Ansprüche 14 oder 15, **dadurch gekennzeichnet, dass** die Aminverbindung aus Aminpolymeren, insbesondere mit einem durch Größenausschlusschromatographie bestimmten gewichtsmittleren Molekulargewicht im Bereich von 500 bis 1.000.000, bevorzugt im Bereich von 500 bis 500.000 und bevorzugt im Bereich von 500 bis 100.000 ausgewählt ist.

**19.** Verfahren nach dem vorhergehenden Anspruch, **dadurch gekennzeichnet, dass** es sich bei der Aminverbindung um ein Aminpolymer handelt, das aus Poly(($C_2$-$C_5$)alkyleniminen) und insbesondere Polyethyleniminen und Polypropyleniminen, insbesondere Poly(ethylenimin)en; Poly(allylamin); Polyvinylaminen und Copolymeren davon, insbesondere mit Vinylamiden; Vinylamin/Vinylformamid-Copolymeren; Polyaminosäuren mit $NH_2$-Gruppen, wie Polylysin; Aminodextran; Aminopolyvinylalkohol, Copolymeren auf Basis von Acrylamidopropylamin; Chitosanen; Polydimethylsiloxanen mit primären Amingruppen am Kettenende oder an Seitenketten, beispielsweise Aminopropyl-Endgruppen oder -Seitengruppen, wie beispielsweise denjenigen der Formel (A) oder (B) oder (C) :

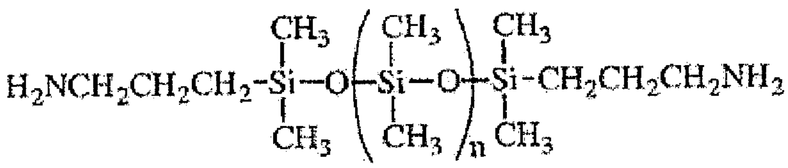

(A)

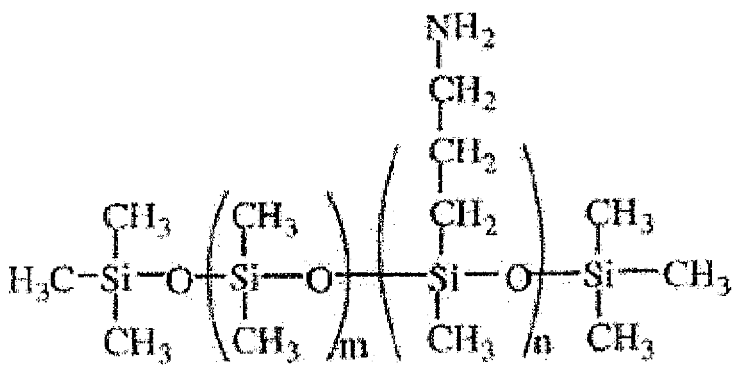

(B)

$$H_2NCH_2CH_2CH_2\text{-}Si(CH_3)_2\text{-}O\text{-}[Si(CH_3)_2\text{-}O]_n\text{-}Si(CH_3)_2C_4H_9 \qquad (C)$$

wobei:

in der Formel (A): der Wert von n so beschaffen ist, dass das gewichtsmittlere Molekulargewicht des Silikons zwischen 500 und 55.000 liegt;
in der Formel (B) die Werte von n und m so beschaffen sind, dass das gewichtsmittlere Molekulargewicht des Silikons zwischen 1000 und 55.000 liegt;
in der Formel (C) der Wert von n so beschaffen ist, dass das gewichtsmittlere Molekulargewicht des Silikons zwischen 500 und 3000 liegt;

Aminodimethiconen der Formel (D):

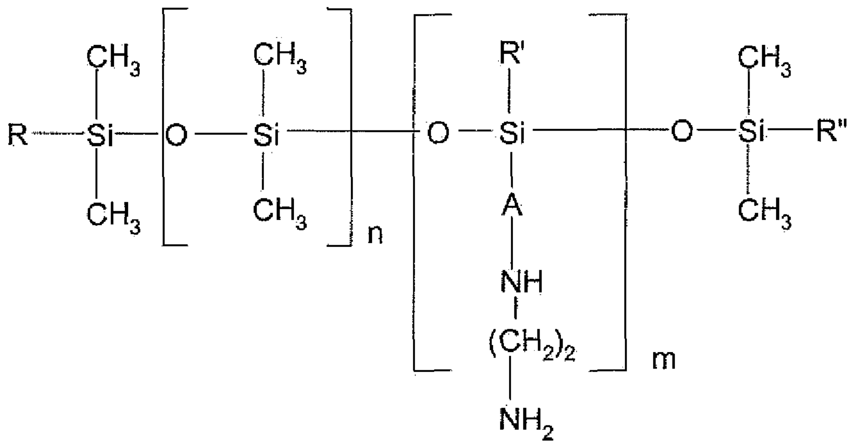

(D)

in der R, R' und R" gleich oder verschieden sind und jeweils für eine $C_1$-$C_4$-Alkyl- oder Hydroxylgruppe stehen, A für eine $C_3$-Alkylengruppe steht und m und n so beschaffen sind, dass das gewichtsmittlere Molekulargewicht der Verbindung zwischen ungefähr 5000 und 500.000 liegt;
Polyetherdiaminen und insbesondere Polyethylenglykol- und/oder Polypropylenglykol-$\alpha,\omega$-diamin; Polytetrahydrofuran-$\alpha,\omega$-diamin (oder Polytetramethylenglykol-$\alpha,\omega$-diamin) und Polybutadien-$\alpha,\omega$-diaminen; Polyamidoamin-Dendrimeren mit endständigen Aminfunktionen;
Pöly(meth)acrylateh oder Poly(meth)acrylamiden mit seitenständigen primären oder sekundären Aminfunktionen wie Poly(3-aminopropyl)methacrylamid oder Poly(2-aminoethyl)methacrylat;
und bevorzugt Polyethylenglykol- und/oder Polypropylenglykol-$\alpha,\omega$-diamin und Polydimethylsiloxanen mit Aminopropyl-Endgruppen
ausgewählt ist.

20. Verfahren nach einem der Ansprüche 14 bis 19, **dadurch gekennzeichnet, dass** die Aminverbindung gemäß einem Molverhältnis von Aminverbindung zu Phosphonsäure im Bereich von 0,01 bis 10, vorzugsweise im Bereich von 0,1 bis 5, bevorzugt im Bereich von 0,1 bis 2 und weiter bevorzugt im Bereich von 0,1 bis 1 verwendet wird.

21. Verfahren nach einem der Ansprüche 14 und 15, **dadurch gekennzeichnet, dass** es sich bei der zusätzlichen Komponente um einen Ton handelt, der aus Tonen der Smektit-Familie wie Laponit und Montmorillonit, der Kaolinit-Familie wie Kaolinit, Dickit, Nacrit, gegebenenfalls modifizierten Tonen der Halloysit-, Dombassit-, Antigorit-, Behthierin-, Pyrophyllit-, Montmorillonit-, Beidellit-, Vermiculit-, Talk-, Stevensit-, Hectorit-, Bentonit-, Saponit-, Chlorit-, Sepiolith- und Illit-Familie ausgewählt ist und vorzugsweise aus Kaolinit, Smektiten wie Laponit und Montmorillonit, Bentonit und Saponit ausgewählt ist.

22. Verfahren nach einem der Ansprüche 14 und 15, **dadurch gekennzeichnet, dass** es sich bei der zusätzlichen Komponente um ein Salz von zweiwertigen oder dreiwertigen Metallionen, das aus Salzen von Ionen, die sich von Al(III), Ca(II), Cu(II), Fe(II), Fe(III), Mg(II), Mn(II), Zn(II) und Mischungen davon ableiten, ausgewählt ist, handelt.

23. Verfahren nach einem der Ansprüche 14 und 15, **dadurch gekennzeichnet, dass** es sich bei der zusätzlichen Komponente um ein Metalloxid, das aus Titandioxid, Eisenoxiden, Zirconiumoxiden, Zinkoxiden, Ceroxiden und Chromoxiden ausgewählt ist, handelt.

24. Verfahren nach einem der Ansprüche 14 und 17 bis 23, **dadurch gekennzeichnet, dass** man auf die Keratinfasern, insbesondere auf die Haut, eine weniger als 5 Minuten vor dem Aufbringen auf die Keratinfasern, insbesondere auf die Haut, hergestellte extemporale Mischung der Zusammensetzung nach einem der Ansprüche 8 bis 11 und der zusätzlichen Komponente oder einer Zusammensetzung, die diese enthält und ein physiologisch unbedenkliches Medium umfasst, aufbringt.

25. Verfahren nach einem der Ansprüche 15 bis 24, **dadurch gekennzeichnet, dass** man auf die Keratinmaterialien, insbesondere auf die Haut, zunächst die Zusammensetzung nach einem der Ansprüche 8 bis 11 aufbringt und dann die zusätzliche Komponente oder eine Zusammensetzung, die diese enthält und ein physiologisch unbedenkliches Medium umfasst, aufbringt.

**26.** Verfahren nach einem der Ansprüche 15 bis 24, **dadurch gekennzeichnet, dass** man auf die Keratinmaterialien, insbesondere auf die Haut, zunächst die zusätzliche Komponente oder eine Zusammensetzung, die diese enthält und ein physiologisch unbedenkliches Medium umfasst, aufbringt und dann die Zusammensetzung nach Anspruch 8 oder 9 aufbringt.

**27.** Verfahren nach einem der Ansprüche 12 bis 26, **dadurch gekennzeichnet, dass** die Zusammensetzung ein Öl umfasst.

**28.** Verfahren nach einem der Ansprüche 12 bis 27, die zur Pflege der Haut, speziell der Gesichtshaut, insbesondere von faltiger Haut, vorgesehen ist.

**29.** Verfahren nach einem der Ansprüche 12 bis 27, **dadurch gekennzeichnet, dass** sie zur Abschwächung von Falten vorgesehen ist.

**30.** Kosmetische Verwendung eines Phosphonsäurepolymers gemäß einem der Ansprüche 1 bis 7 und gegebenenfalls in Abmischung mit einer zusätzlichen Verbindung gemäß einem der Ansprüche 14 bis 19 und 21 bis 23 als Straffungsmittel für die Haut, insbesondere für faltige Haut.

**31.** Zusammensetzung, erhalten durch Mischen einer Zusammensetzung nach einem der Ansprüche 8 bis 11 und einer zusätzlichen Komponente gemäß einem der Ansprüche 14 bis 19 und 21 bis 23 oder einer Zusammensetzung, die diese enthält und ein physiologisch unbedenkliches Medium umfasst.

**32.** Kit, umfassend eine erste Zusammensetzung gemäß einem der Ansprüche 8 bis 11 und eine zweite Zusammensetzung, die eine zusätzliche Verbindung gemäß einem der Ansprüche 14 bis 19 und 21 bis 23 enthält und ein physiologisch unbedenkliches Medium umfasst, wobei die erste Zusammensetzung und die zweite Zusammensetzung jeweils in einer separaten Verpackungsanordnung verpackt sind.

## Claims

**1.** Copolymer resulting from the polymerization of:

(i) 70% to 95% by weight, of the total weight of the monomers, of isobornyl (meth)acrylate;
(ii) 1% to 30% by weight of vinylphosphonic acid monomer of formula (I):

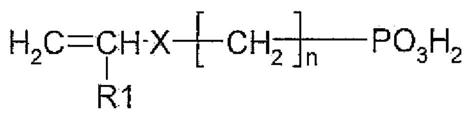

(I)

in which:

R1 denotes H or $-CH_3$;
X denotes a covalent bond and n denotes an integer ranging from 0 to 14;
or X denotes a -COO- group and n denotes an integer ranging from 2 to 6;

(iii) 0 to 20% by weight of an additional monomer which is different from the monomers of isobornyl (meth)acrylate and of vinylphosphonic acid.

**2.** Copolymer according to Claim 1, **characterized in that**, for monomer (I):
X denotes a covalent bond and n is an integer ranging from 0 to 6 or X denotes a -COO- group and n is an integer ranging from 2 to 4.

**3.** Copolymer according to either of the preceding claims, **characterized in that**, for monomer (I), R1 = H and X denotes a covalent bond and n is an integer ranging from 0 to 4.

**4.** Copolymer according to one of the preceding claims, **characterized in that** monomer (I) is chosen from:

vinylphosphonic acid;
3-butenylphosphonic acid;
4-pentenylphosphonic acid;
10-undecenylphosphonic acid;
11-dodecenylphosphonic acid;
2-phosphonoethyl ester of 2-methyl-2-propenoic acid;
2-phosphonoethyl ester of 2-propenoic acid;
and preferably vinylphosphonic acid.

**5.** Copolymer according to one of the preceding claims, **characterized in that** the additional monomer is a monomer of formula (II):

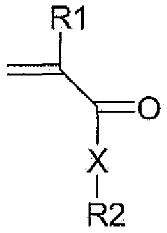

(II)

in which:

$R_1$ denotes a hydrogen atom or a methyl radical;
X denotes O or NH or $NR_3$;
$R_2$ denotes a linear $C_1$-$C_{22}$ or branched $C_3$-$C_{10}$ or cyclic $C_5$-$C_7$ alkyl radical, or a linear $C_3$-$C_{20}$ or branched $C_6$-$C_{20}$ or cyclic $C_5$-$C_7$ unsaturated hydrocarbon-based radical, or an $-(Si(CH_3)_2O)_b-CH_3$ radical, with b ranging from 5 to 70, it being understood that X = O when $R_2$ is a radical of formula $-(Si(CH_3)_2O)_b-CH_3$;
$R_3$ denotes a linear $C_1$-$C_{12}$ or branched $C_3$-$C_{12}$ alkyl radical.

**6.** Copolymer according to one of the preceding claims, **characterized in that** it results from the polymerization of:

- 70% to 95% by weight, of the total weight of the monomers, of isobornyl (meth)acrylate;
- 5% to 30% by weight of vinylphosphonic acid;

and preferably of:

- 75% to 95% by weight, of the total weight of the monomers, of isobornyl (meth)acrylate;
- 5% to 25% by weight of vinylphosphonic acid.

**7.** Copolymer according to any one of the preceding claims, **characterized in that** it is chosen from 80/20 or 90/10 (weight/weight) isobornyl acrylate / vinylphosphonic acid copolymers.

**8.** Composition comprising, in a physiologically acceptable medium, a copolymer according to any one of the preceding claims.

**9.** Composition according to the preceding claim, **characterized in that** the copolymer is present in a content ranging from 0.1% to 10% by weight, relative to the total weight of the composition, preferably from 0.5% to 10% by weight of active material, preferentially ranging from 1% to 8% by weight, and more preferentially ranging from 1% to 6% by weight.

**10.** Composition according to either of Claims 8 and 9, **characterized in that** it comprises a colorant.

**11.** Composition according to the preceding claim, **characterized in that** the colorant is chosen from pigments.

**12.** Process for caring for or making up keratin materials, comprising the topical application to the keratin materials of a composition according to one of Claims 8 to 11.

**13.** Process for making up keratin materials, comprising the topical application to the keratin materials of a composition according to either of Claims 10 and 11.

**14.** Process according Claim 12 or 13, **characterized in that** the topical application, to keratin materials, of an extemporaneous mixture of the composition according to one of Claims 8 to 11 and of an additional component chosen from:

(i) an amine compound chosen from amine compounds bearing several primary amine and/or secondary amine groups and aminosilanes,
(ii) salts of divalent or trivalent metal ions,
(iii) clays,
(iv) metal oxides,

or of a composition containing same and comprising a physiologically acceptable medium, is carried out.

**15.** Process according to either of Claims 12 and 13, **characterized in that** the sequential application, to keratin materials, of the composition according to one of Claims 8 to 11 and of an additional component chosen from:

(i) an amine compound chosen from amine compounds comprising several primary amine and/or secondary amine groups and aminosilanes,
(ii) salts of divalent or trivalent metal ions,
(iii) clays,
(iv) metal oxides,

or of a composition containing same and comprising a physiologically acceptable medium, is carried out.

**16.** Process according to either one of Claims 14 and 15, **characterized in that** the amine compound comprises from 2 to 20 carbon atoms.

**17.** Process according to any one of Claims 14 to 16, **characterized in that** the amine compound is chosen from N-methyl-1,3-diaminopropane, N-propyl-1,3-diaminopropane, N-isopropyl-1,3-diaminopropane, N-cyclohexyl-1,3-diaminopropane, 2-(3-aminopropylamino)ethanol, 3-(2-aminoethyl)aminopropylamine, bis(3-aminopropyl)amine, methylbis(3-aminopropyl)amine, N-(3-aminopropyl)-1,4-diaminobutane, N,N-dimethy1dipropylenetriamine, 1,2-bis(3-aminopropylamino)ethane, N,N'-bis(3-aminopropyl)-1,3-propanediamine, ethylenediamine, 1,3-propylenediamine, 1,4-butylenediamine, lysine, cystamine, xylenediamine, tris(2-aminoethyl)amine and spermidine; and the aminosilanes of formula (III):

$$R'_1Si(OR'_2)_z(R'_3)_x \qquad (III)$$

in which:

• $R'_1$ is a linear or branched, saturated or unsaturated, cyclic or acyclic $C_1$-$C_6$ hydrocarbon-based chain substituted with a group chosen from the following groups:

- amine $NH_2$ or NHR with R = $C_1$-$C_4$ alkyl,
- an aryl or aryloxy group substituted with an amino group or with a $C_1$-$C_4$ aminoalkyl group,
$R'_1$ possibly being interrupted in its chain with a heteroatom (O, S, NH) or a carbonyl group (CO), $R'_1$ being linked to the silicon atom directly via a carbon atom,

• $R'_2$ and $R'_3$, which may be identical or different, represent a linear or branched alkyl group comprising from 1 to 6 carbon atoms,
• z denotes an integer ranging from 1 to 3, and
• x denotes an integer ranging from 0 to 2,

with

$$z + x = 3;$$

and preferably chosen from ethylenediamine, lysine and 3-aminopropyltriethoxysilane, and is preferably 3-aminopropyltriethoxysilane.

**18.** Process according to either of Claims 14 and 15, **characterized in that** the amine compound is chosen from amine-based polymers, in particular having a weight-average molecular weight, determined by size exclusion chromatography, ranging from 500 to 1 000 000, preferably ranging from 500 to 500 000, and preferentially ranging from 500 to 100 000.

**19.** Process according to the preceding claim, **characterized in that** the amine compound is an amine-based polymer chosen from poly (($C_2$-$C_5$)alkyleneimines), and in particular polyethyleneimines and polypropyleneimines, in particular poly(ethyleneimine)s; poly(allylamine); polyvinylamines and copolymers thereof, in particular with vinylamides; vinylamine/vinylformamide copolymers; polyamino acids bearing $NH_2$ groups, such as polylysine; aminodextran; amino polyvinyl alcohol, acrylamidopropylamine-based copolymers; chitosans;
polydimethylsiloxanes comprising primary amine groups at the chain end or on side chains, for example aminopropyl side or end groups, for instance those of formula (A) or (B) or (C):

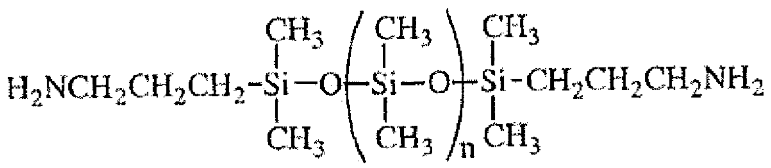

(A)

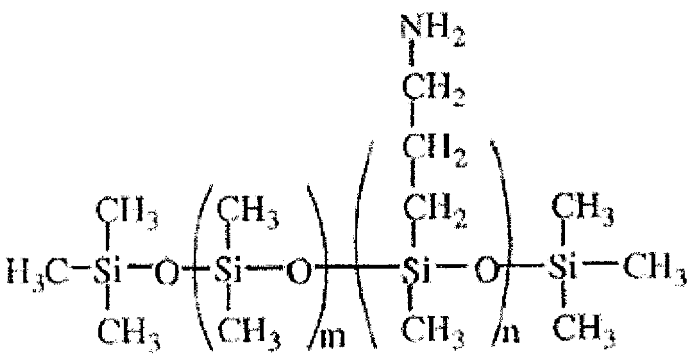

(B)

$H_2NCH_2CH_2CH_2$-$Si(CH_3)_2$-O-$[Si(CH_3)_2$-$O]_n$-$Si(CH_3)_2C_4H_9$ (C)

with:

in formula (A): the value of n is such that the weight-average molecular weight of the silicone is between 500 and 55 000.
in formula (B), the values of n and m are such that the weight-average molecular weight of the silicone is between 1000 and 55 000;
in formula (C), the value of n is such that the weight-average molecular weight of the silicone is between 500 and 3000;

amodimethicones of formula (D):

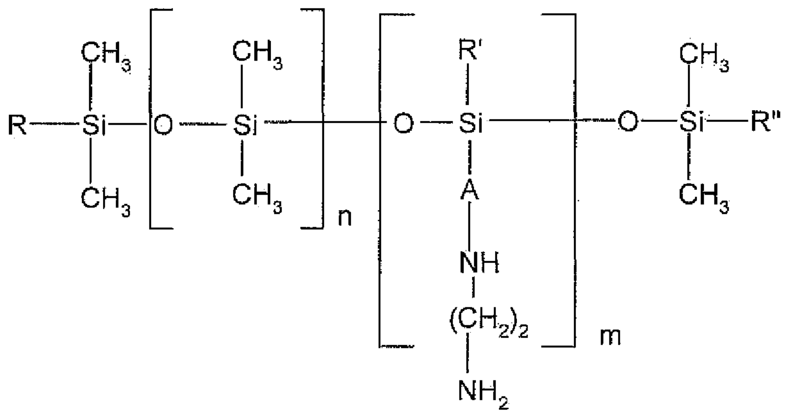

(D)

in which R, R' and R", which may be identical or different, each represent a $C_1$-$C_4$ alkyl or hydroxyl group, A represents a $C_3$ alkylene group and m and n are such that the weight-average molecular mass of the compound is between 5000 and 500 000 approximately; polyetherdiamines and in particular polyethylene glycol and/or polypropylene glycol α,ω-diamines; polytetrahydrofuran (or polytetramethylene glycol) α,ω-diamines and polybutadiene α,ω-diamines ; polyamidoamine dendrimers bearing amine end functions; poly(meth)acrylates or poly(meth)acrylamides bearing primary or secondary amine side functions, such as poly (3-aminopropyl)methacrylamide or poly (2-aminoethyl) methacrylate;
and preferably polyethylene glycol and/or polypropylene glycol α,ω-diamines and polydimethylsiloxanes comprising aminopropyl end groups.

**20.** Process according to any one of Claims 14 to 19, **characterized in that** the amine compound is used according to an amine compound / phosphonic acid mole ratio ranging from 0.01 to 10, preferably ranging from 0.1 to 5, preferentially ranging from 0.1 to 2 and more preferentially ranging from 0.1 to 1.

**21.** Process according to either one of Claims 14 and 15, **characterized in that** the additional component is a clay chosen from clays of the smectite family, such as laponite and montmorillonite, of the kaolinite family, such as kaolinite, dickite, nacrite, optionally modified clays of the halloysite, dombassite, antigorite, benthierine, pyrophyllite, montmorillonite, beidellite, vermiculite, talc, stevensite, hectorite, bentonite, saponite, chlorite, sepiolite and illite family; and preferably chosen from kaolinite, smectites such as laponite and montmorillonite, bentonite and saponite.

**22.** Process according to either one of Claims 14 and 15, **characterized in that** the additional component is a salt of divalent or trivalent metal ions chosen from salts of ions derived from Al(III), Ca(II), Cu(II), Fe(II), Fe(III), Mg(II), Mn(II), Zn(II) and mixtures thereof.

**23.** Process according to either one of Claims 14 and 15, **characterized in that** the additional component is a metal oxide chosen from titanium dioxide, iron oxides, zirconium oxides, zinc oxides, cerium oxides and chromium oxides.

**24.** Process according to any one of Claims 14 and 17 to 23, **characterized in that** an extemporaneous mixture prepared less than 5 minutes before application to keratin materials, in particular to the skin, of the composition according to one of Claims 8 to 11 and of said additional component, or of a composition containing same and comprising a physiologically acceptable medium, is applied to keratin materials, in particular to the skin.

**25.** Process according to any one of Claims 15 to 24, **characterized in that** the composition according to one of Claims 8 to 11 is first applied to keratin materials, in particular to the skin, then said additional component or a composition containing same and comprising a physiologically acceptable medium is applied.

**26.** Process according to any one of Claims 15 to 24, **characterized in that** said additional component, or a composition containing same and comprising a physiologically acceptable medium, is first applied to keratin materials, in particular to the skin, then the composition according to Claim 8 or 9 is applied.

**27.** Process according to any one of Claims 12 to 26, **characterized in that** the composition comprises an oil.

**28.** Process according to any one of Claims 12 to 27, intended for caring for the skin, more particularly facial skin, in

particular wrinkled skin.

**29.** Process according to any one of Claims 12 to 27, **characterized in that** it is intended for attenuating wrinkles.

**30.** Cosmetic use, as a skin tensioning agent, in particular a wrinkled-skin tensioning agent, of a phosphonic polymer as defined in any one of Claims 1 to 7 and optionally as a mixture with an additional compound as defined in one of Claims 14 to 19 and 21 to 23.

**31.** Composition obtained by mixing a composition according to one of Claims 8 to 11 and an additional component as defined in one of Claims 14 to 19 and 21 to 23, or a composition containing same and comprising a physiologically acceptable medium.

**32.** Kit comprising a first composition according to one of Claims 8 to 11 and a second composition comprising an additional compound as defined in one of Claims 14 to 19 and 21 to 23 and comprising a physiologically acceptable medium, the first and second compositions each being packaged in a separate packaging assembly.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1776946 A
- EP 1621560 A